# EUROPEAN PATENT SPECIFICATION

(11) **EP 3 841 442 B1**
(45) Date of publication and mention of the grant of the patent: **12.06.2024**
(21) Application number: 19779013.2
(22) Date of filing: 30.09.2019
(51) Int. Cl.: G05B 19/418, G06Q 10/06, G06Q 50/04, G06Q 10/0639

(54) **MONITORING SYSTEM AND METHOD FOR BIOPHARMACEUTICAL PRODUCTS**
ÜBERWACHUNGSSYSTEM UND -VERFAHREN FÜR BIOPHARMAZEUTISCHE PRODUKTE
SYSTÈME ET PROCÉDÉ DE SURVEILLANCE POUR PRODUITS BIOPHARMACEUTIQUES

(30) Priority: 24.08.2018 IN 201841031732
(43) Date of publication of application: 30.06.2021
(60) Divisional application: 24164040.8
(73) Proprietor: Cytiva Sweden AB, 751 84 Uppsala (SE)
(72) Inventor: GEBAUER, Klaus, 75184 Uppsala (SE); LAKHANI, Hanish, Bangalore, Karnataka 56006 (IN); PURANIK, Swapnil, Bangalore, Karnataka 56006 (IN); GARG, Nishu, Bangalore, Karnataka 56006 (IN); J, Hemalatha, Bangalore, Karnataka 56006 (IN)
(74) Representative: Wu, Ping
(86) International application number: PCT/EP2019/076485
(87) International publication number: WO 2020/039104

(56) References cited:
- WO-A2-2014/140746
- US-B1- 9 591 273
- YANG JUN ET AL: "Vision-based action recognition of construction workers using dense trajectories", ADVANCED ENGINEERING INFORMATICS, ELSEVIER, AMSTERDAM, NL, vol. 30, no. 3, 13 May 2016 (2016-05-13), pages 327-336, XP029688234, ISSN: 1474-0346, DOI: 10.1016/J.AEI.2016.04.009

## Description

### FIELD OF THE INVENTION

The present disclosure relates to a system for monitoring manufacture of a selected biopharmaceutical product.

The present disclosure further relates to a method for monitoring a set-up for manufacture and/or setting up for manufacture of a selected one among a plurality of biopharmaceutical products.

### BACKGROUND OF THE INVENTION

Industrial monitoring systems for use outside of the biopharmaceutical industry are generally known. See, for example, US 9,591,273 B1, WO 2014/140746 A2 and YANG JUN ET AL, "Vision-based action recognition of construction workers using dense trajectories", ADVANCED ENGINEERING INFORMATICS, ELSEVIER, AMSTERDAM, NL, (20160513), vol. 30, no. 3, doi:10.1016/J.AEI.2016.04.009, ISSN 1474-0346, pages 327 - 336, XP029688234 [A], page 330.

However, the past decade has seen a significant shift in the nature of the products being manufactured and sold by the innovative biopharmaceutical industry. The global biopharmaceutical portfolio of today reflects a greater prevalence of large molecule drugs, expansion in the number of personalized or targeted products, and a rise of treatments for many orphan diseases. These development trends provide for biopharmaceutical products with extremely limited production runs, highly specific manufacturing requirements, and genotype-specific products. The on-going shift in the product mix provides a need for continuous improvement of the efficiency and effectiveness of production biopharmaceutical manufacturing.

Biologic medicines (also called biologics) such as for example therapeutic proteins, monoclonal antibodies, vaccines etc., are complex molecules made by or from living cells. They often require parenteral administration by infusion or injection, thereby requiring highly specialized manufacturing, special storage and handling, and a tightly controlled, high quality manufacturing and distribution network to ensure safety and effectiveness. Developments are also observed with regard to orphan drugs, i.e., drugs aimed at diseases with patient populations of under 200,000, and there has been a steady increase over the past decade. Furthermore, manufacturers are increasingly focusing on more complex diseases for which there are few or no effective treatments. New treatments for these diseases are characterized by small volume products.

Another important trend within biopharmaceuticals is the emergence of personalized medicine; products that target a specific population of patients. Over time, as patient-level personalized medicines are introduced, manufacturing and product supply complexity will likely increase.

The biopharmaceutical products may be intended for therapeutic or diagnostic use, for example. To ensure adequate product quality as well as well controlled and auditable production conditions, cGMP (current Good Manufacturing Practices) and QMS (Quality Management Systems) are typically required and applied. GMP processing environments are designed to conform to the guidelines recommended by agencies that control the authorization and licensing of the manufacture and sale of pharmaceutical products, such as for example the FDA (Food and Drug Administration).

These drug portfolio trends have contributed to an increase in the number and complexity of products being manufactured and sold commercially. In addition, above trends and the rapid market growth of biologic therapies also implies a drastically increasing number of small production batches during drug development before product reach the market, specifically for production of clinical trial material. As the drug development process generally is characterized as a 'development funnel' with a significantly larger number of drug candidates going through clinical trials than the number of successful and eventually approved drugs. Drug substance production during clinical trials, as well as during regular production, requires for high safety and throughput provided by manufacturing systems. However, drug substance production for clinical trials generally requires even higher flexibility in manufacturing methods and systems to adapt to different requirements and production protocols yet complying with rigorous cGMP and QMS requirements. With the background of all biopharma products being subjected to cost pressure, cost reductions during clinical manufacturing and drug development are equally important as for regular production of approved drugs. Cost efficient and safe production systems providing high throughput therefore need to accommodate frequent process changes, process and equipment reconfigurations and other modifications. Additionally, some of the new medicines have increased the need for more complex manufacturing processes, and more advanced equipment. Continuous and connected processing regimes are becoming additions or alternatives to traditional batch manufacturing methods and may have advantages in terms of overall product and/or process quality, efficiency and throughput or cost.

Overall, these drug portfolio trends indicate that there is a need for improved manufacturing creating flexibility with uncompromised quality, while creating operating efficiencies that can help reduce costs. One recent technological development to reduce production cost, increase production throughput and quality as well as reduce safety concerns is the use of single-use technology (SUT) for processing. With single-use technology equipment, wetted parts that are in contact with the process fluid and drug product during processing, such as for example fluid storage vessels, tubing, separation equipment etc., are provided as consumables which are to be installed and used for a specific process only and are disposed thereafter. SUT consumables are typically produced, configured and packaged in clean room environments, and pre-sterilized (e.g. by gamma irradiation) prior to use in the biomanufacturing process. In contrast to using the traditional and fixed installations of stainless steel tubing and stainless steel reactors and vessels, SUT equipment and consumables provide great flexibility in adapting to different process scenarios and configurations, simply by re-arranging (movable) equipment, installing and replacing different consumables over processes. For example, a tank bin can be fitted with a clean and/or sterile SUT fluid bag to provide clean and contained enclosure for fluid and its processing. The advantage of using single-use technology (SUT) fluid handling equipment is primarily that cross-contamination in between production batches and campaigns is eliminated when the SUT equipment is used for a single drug product only. The SUT equipment is disposed of after use, which can be after a single run, batch or campaign comprising multiple runs and batches. When providing SUT equipment pre-sterilized or bioburden controlled, initial cleaning and sanitization (for example by contacting the flow path with sodium hydroxide solutions) or sterilization can be avoided. When using the SUT for a single run or batch only, even cleaning post-use may be omitted. With these features, SUT equipment provides improved efficiency, safety and convenience. Nowadays, SUT equipment is available for the majority of all types of equipment and/or unit operations, among them bioreactors for cell culture or fermentation, buffer bags for liquid storage, tubing and pumps for liquid transfer and filling operations, filters, chromatography columns and related systems for separations. However, the adaption to single-use technology also implies a higher throughput and flow of materials, i.e. SUT consumables, in production processes and facilities compared to traditional manufacturing with fixed stainless steel installations. Further, a significant number of additional operational steps and operator interactions (work) is required to handle said material flow as well as to install and removed the single-use consumables prior and after processing, as well as documenting material, material flow and their use in the processing. The frequent change associated with SUT consumables implies that new (fresh) installations of the processing lines are to be used and documented for each drug run, batch or campaign in a manufacturing process. The biopharma industry is rapidly adopting SUT for above mentioned reasons, however, this adaption is also characterized by least some of the following challenges:
- Frequent installation of complete fluid paths required
- Large number of material to be handled by operator and managed in planning, logistics and documentation
- Materials (i.e. consumables and commodity articles) are subject to change over processes due to sourcing variability and/or lack of standardization in single-use consumables
- Many manual interaction steps

### SUMMARY OF THE INVENTION

An object of the present disclosure is to provide solutions, which seek to mitigate, alleviate, or eliminate one or more of the above-identified deficiencies in the art and to provide improved biopharmaceutical manufacturing.

This object is obtained by a monitoring system for monitoring manufacture of a selected biopharmaceutical product, as defined by the appended claims. The monitoring system comprises a manufacture system with capability to manufacture a plurality of biopharmaceutical products, or to manufacture a class of biopharmaceuticals in a plurality of pre-defined protocols or production scenarios, which may differ in their specific operating conditions, equipment/unit operation setup or processing regimes, for example batch or continuous and connected processing. A unit operation is a defined processing step, such as a reactor, a filtration step, a chromatography operation, etc. The monitoring system comprises at least one image capture device arranged to capture a scene comprising the manufacture system, and a processing element connected to said at least one image capture device and arranged to process images captured by said at least one image capture device to track operator interactions and/or a result of operator interactions within the scene. The processing element is further arranged to compare a predefined workflow process scheme relating to the selected biopharmaceutical product to at least a part of the tracked operator interactions with the manufacture system and/or to at least a part of the result of the tracked operator interactions with the manufacture system. The processing element is further arranged to determine whether at least one pre-set criterion set by the workflow process scheme is fulfilled based on the comparison.

The use of the predefined workflow process scheme associated to the specific selected biopharmaceutical product and the comparison with at least a part of tracked user operator interactions with the manufacture system and/or to at least a part of the result of the tracked operator interactions with the manufacture system enables improved manufacturing creating flexibility with uncompromised quality, while creating operating efficiencies that can help reduce costs. The quality is herein applied to a plurality of or all parts of the process, such as verification before installation, i.e. verification of a so called Bill Of Material, BOM, comprising material needed for accomplishing an installation set-up, installation of an installation set-up, the installation procedure, approval/verification of the installation set-up and installation procedure, processing itself, and teardown of the installation set-up.

All parts above may be comprised in the predefined workflow process scheme above. Verification and documentation may be obtained. Thus, the predefined workflow process scheme comprises at least the data of a traditional batch protocol but it may further comprise other processes, i.e. an extended batch protocol as is apparent from the above. The predetermined workflow process scheme may also comprise the data of batch records, thus, data relating to approval and verification of the batch protocol/extended batch protocol.

Thus, the terms 'Batch record' (BR, eBR - electronic Batch Record) as well as Batch protocol are equivalent to "predefined workflow process scheme" The protocol is the instruction and the record is the completed protocol as documentation and result of an executed process.

The monitoring system as disclosed herein supports (and refines) electronic protocols and records, thereby allowing the processing element to could not communicate and be effective in its flexibility, agility, adaptability and learning capability.

A deviation from an installation set-up as indicated by the predetermined scheme may be detected. The installation set-up may comprise the verification before installation (verification of so called Bill Of Material) and/or installation of the installation set-up and/or teardown of the installation set-up. Deviations from the installation set-up adversely affecting the final biopharmaceutical product may lead to a determination that the manufacture system is not correctly set up.

Further, a deviation from the installation procedure as indicated by the predetermined scheme may be detected. Deviations from the installation procedure adversely affecting the final biopharmaceutical product may lead to a determination that the manufacture system is not correctly set up.

Further, deviations from the installation set-up and/or installation procedure known to adversely affect the final biopharmaceutical product and other deviations may then be recorded. Characteristics of the final biopharmaceutical product so manufactured may be recorded in association with the recorded deviations. A detailed analysis of the impact of different procedure deviations may then be obtained, which in turn may be used to further improve the installation procedure of the predetermined scheme. Accordingly, deviations in the final biopharmaceutical product so produced may even be decreased with time.

The monitoring system and its way to work with electronic work instructions also allows for providing a certain flexibility in using different external devices (such as consumables) that result in the same functionality of the assembled final system. The monitoring system allows for guiding the operator through this according to the electronic batch protocol. Hereby, flexibility is tackling material sourcing variability; the system can be self learning and improve instructions and autonomy in electronic verification during change and repetitive use of new configurations.

Further aspects and embodiments of the present invention are defined in the dependent claims.

In addition to the advantages mentioned above, which of course also are applicable to the method and computer program product embodiments, the disclosure provides the advantage of improved process robustness and the possibility for accelerated scale-up to commercial production of biopharmaceutical products. Further advantages include increased flexibility and reduced production lead times.

### BRIEF DESCRIPTION OF THE DRAWINGS

Fig 1 is a block scheme illustrating an example of a monitoring system.
Fig 2 is a block scheme illustrating an example of a user interface suitable in a monitoring system of fig 1.
Fig 3 illustrates schematically an example of a production record for a selected biopharmaceutical product.
Fig 4 illustrates schematically an example of a manufacture facility scene.
Fig 5 illustrates schematically an example of a time line for recognition of external devices and other equipment within a facility.
Fig 6 is a flow chart illustrating an example of a method for monitoring a set-up for manufacture and/or setting up for manufacture of a selected one among a plurality of biopharmaceutical products.
Fig 7 illustrates a scheme illustrating different levels of providing manufacture support aligned with the ISA95 standard.
Fig 8 illustrates an example of a time line over high level work flow operations and activities and instructions associated thereto.

### DETAILED DESCRIPTION

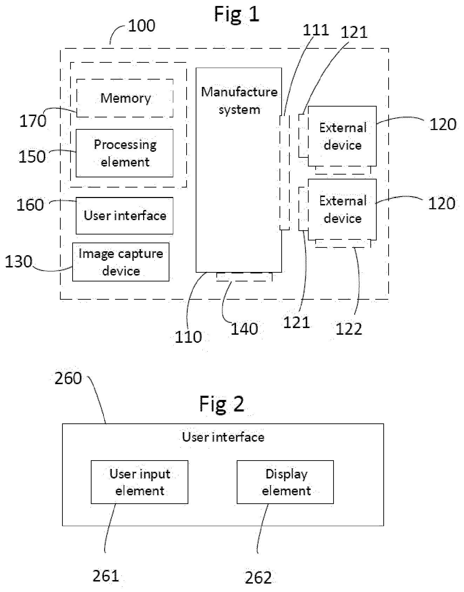

Fig 1 discloses a system 100 for monitoring manufacture of a selected pharmaceutical product.

When it comes to manufacture of biopharmaceutical products, the process is important to the final biopharmaceutical product, both with regard to the processing setup in terms of type, configuration and installation of the manufacture system and with regard to detailed processing regimes in terms of operating parameters. Therefore, the installation procedure for setting up for manufacture may have an impact on the final biopharmaceutical product, as wrong or incomplete installations may cause fluid leakage, malfunction or alteration of processing steps and their outcome. Regulatory and/or legal requirements for production of biopharmaceuticals, such as approval by the FDA (food and drug administration), require rigorous control and documentation of set-up, installation, and use of equipment, for example with regard to operator interaction and automated process control. Operating procedures, such as batch protocols, and records, such as batch records, are a fundamental concept in development and production of biopharmaceuticals including approval and monitoring from regulatory bodies. The system as defined herein is useful among others in monitoring set-up of an installation for manufacture as well as procedures for setting up the installation for manufacture.

The monitoring system is adapted for manufacture of a variety of biopharmaceutical products. The monitoring system is adapted for handling small-volume runs, which require frequent changeovers and may necessitate equipment reconfigurations and updates.

Further, as stated above, the system is adapted for handling manufacture using manufacturing processes with increased complexity and/or more advanced equipment may be used.

The system allows for improved manufacturing creating flexibility with uncompromised quality, while creating operating efficiencies that can help reduce costs.

The monitoring system 100 comprises a manufacture system 110. The manufacture system is adapted for manufacture of the selected one among a plurality of biopharmaceutical products. Characteristically, the manufacture system is selected based on the biopharmaceutical product to be manufactured. Characteristically the same or similar configuration of the manufacture system can be used for the production of several biopharmaceutical products.

The manufacture system 110 may comprise a liquid chromatography system preferably configured to operate with at least one column and configured for purification of a sample comprising a target product using a predefined process. The manufacture system may comprise one or several manufacture devices. Each manufacture device may be adapted to the pharmaceutical product to be manufactured. Each manufacture device may be provided with installations and configurations to accommodate the outcome of said manufacture devices. Examples for typical manufacture devices are devices for fluid transfer, fluid filling, fluid or buffer preparation, devices for cell culturing or fermentation such as bioreactors, devices for cell harvest or clarification, devices for chromatography or membrane adsorption, filtration or buffer exchange devices etc. Manufacture devices may be combined such that an integrated system running multiple processing steps is achieved.

The manufacture system may instead be defined as comprising one or several unit operations with installations and configurations to accommodate the outcome of said unit operations. Examples for typical unit operations are fluid transfer, fluid or buffer preparation operations, cell culturing operation using bioreactor, cell harvest or clarification operations, chromatography or membrane adsorption operations, filtration or buffer exchange operations etc. The manufacture system may comprise unit operations that are combined such that an integrated system running multiple processing steps is achieved, and procedures, instructions, documentation and operator guidance of the manufacture system may be adapted to this. Further, the manufacture system and its parts may be adapted to batch processing and/or to continuous and connected processing regimes. Thus, the different manufacture devices are arranged to perform different types of unit operations

The monitoring system has Procedures, instructions, documentation and operator guidance of the manufacture system may be adapted to this. Further, the monitoring system and its parts may be adapted to batch processing and/or to continuous and connected processing regimes.

The monitoring system 100 may further comprise a user interface 160. The biopharmaceutical product may then be selected by means of the user interface.

The monitoring system comprises further at least one image capture device 130 arranged to capture a scene comprising the manufacture system. The at least one image capture device may be arranged to capture images of substantially the entire scene.

The at least one image capture device 120 may comprise a camera arranged to record images within the visual field, a thermal camera and/or a three dimensional camera. The camera may be arranged to generate a time series of images. At least one of the cameras may be a video camera. At least a part of the images may be time stamped.

Additional sensor elements may be provided to obtain additional position information for example as a distance or a coordinate. The sensor element may comprise radar and/or ladar and/or a GPS receiver etc. The additional sensor element(s) may be arranged at the image capturing device or in a known relation thereto. The additional sensor element(s) may be arranged to provide redundancy and/or additional spatial resolution to the images captured by the image capturing device.

The monitoring system 100 comprises further a processing element 150 connected to said at least one image capture device. The processing element 150 is arranged to process images captured by said at least one image capture device to track operator interactions and/or a result of operator interactions within the scene.

The processing element 150 is further connected to a memory 170. The memory comprises a plurality of workflow process schemes. Each scheme is relating to a specific biopharmaceutical product or product batch. The predetermined workflow process scheme relating to the specific biopharmaceutical product or product batch may comprise one or a plurality of components. The predetermined workflow process scheme associated to the specific selected biopharmaceutical product may comprise components relating to at least a part of tracked user operator interactions with different parts of the manufacture system and/or to at least a part of the result of the tracked operator interactions with the different parts of the manufacture system, such as installation of an installation set-up, the installation procedure, processing itself, and teardown of the installation set-up.

The predetermined workflow process scheme may also comprise processes which may not necessarily be connected to the manufacture system itself, such as verification before installation (verification of so called Bill Of Material).

Thus, the predefined workflow process scheme may comprise the processes and data typically comprised in a traditional batch protocol but it may further comprise other processes and data, i.e. an extended batch protocol as is apparent from the above.

The predetermined workflow process scheme may also comprise approval/verification of the installation set-up and installation procedure. Thus, the predetermined workflow process scheme may also comprise the data of batch records, thus, data relating to approval and verification of the batch protocol/extended batch protocol.

The workflow process schemes and/or components thereof may be permanently stored in the memory or checked in for manufacture of a specific biopharmaceutical product.

The processing element 150 is further is arranged to compare the predefined workflow process scheme relating to a selected biopharmaceutical product to at least a part of the tracked operator interactions with the manufacture system and/or to at least a part of the result of the tracked operator interactions with the manufacture system.

The processing element 150 is further arranged to determine whether at least one pre-set criterion set by the workflow process scheme is fulfilled based on the comparison.

Thereby, a deviation from the installation set-up as indicated by the predetermined scheme may be detected. Deviations from the installation set-up adversely affecting the final biopharmaceutical product may lead to a determination that the manufacture system is not correctly set up.

Further, a deviation from the installation procedure as indicated by the predetermined scheme may be detected. Deviations from the installation procedure adversely affecting the final biopharmaceutical product may lead to a determination that the manufacture system is not correctly set up.

Further, deviations from the installation set-up and/or installation procedure known to adversely affect the final biopharmaceutical product and other deviations may then be recorded. Characteristics of the final biopharmaceutical product so manufactured may be recorded in association with the recorded deviations. A detailed analysis of the impact of different procedure deviations may then be obtained, which in turn may be used to further improve the installation procedure of the predetermined scheme. Accordingly, deviations in the final biopharmaceutical product so produced may even be decreased with time. The analysis may for example be made using machine learning. This will be described more in detail below.

The predetermined workflow process scheme defines in different embodiments a plurality of predetermined interconnections between a plurality of first connector elements 111 of the manufacture system 110 and at least one second connector element 121 of at least one external device 120 connectable to the manufacture system 110. The processing element 150 may then be arranged to process the images and/or information obtained by additional sensor elements or by operator input, to identify interconnections between the first and second connector elements 111, 121 in said images, to compare each identified interconnection with the predetermined interconnections defined by the predetermined workflow process scheme and to determine which operator interactions results fulfil the at least one pre-set criterion based on said comparison. Thus, it is in accordance with these embodiments then determined whether the manufacture system is connected to the respective external device or not. Thus, it is in accordance with these embodiments then determined whether the manufacture system is connected to the respective external device or not. Further, when the manufacture system comprises a plurality of manufacture devices, the same may be applied for interconnections between different manufacture devices of the manufacture system.

The processing element 150 has the ability to guide the operator in avoiding deviations, for example establishing wrong interconnections. When working with single-use consumables, and for example with single-use aseptic connectors, a wrongly established interconnection may not be reversible without a breach in the aseptic or sterile state of the assembly and thereby compromising the quality in the set-up and the production process. The correction of a faulty interconnection in an assembly may therefore require the replacement and re-installation of a larger subassembly or in worst case the complete fluid line.

The processing element 150 has further the ability to guide the operator in correcting and resolving moderate deviations according to pre-determined solutions and actions determined and presented by the processing element, and the processing element 150 may do so in a proactive manner. This increases the overall efficiency in manufacturing as minor deviations dealt with and corrected by help of the monitoring system can result in an approved and verified result compatible with the predetermined protocol. A separate follow up by a supervisor and/or formal investigations may be avoided or at least facilitated by help of actions, corrections and documentation provided by the monitoring system.

The monitoring system may analyse the cause, effect and resolution of deviations to further improve the installation procedure in compliance to the predetermined scheme and/or prevent future deviations. It may also analyse and propose improvements or alternatives of the predetermined scheme. In this respect, an intelligent or learning system may be used. This will be discussed more in detail later.

Accordingly, deviations in the final biopharmaceutical product so produced may even be decreased with time.

The at least one external device 120 may comprise a consumable and/or a sensor element, and/or an electronic device and/or a power supply.

The consumable may be a flow path, for example, comprising wetted fluid lines, bags, connectors and sensors. The consumable is a single-use flow path which may be installed and interchanged for different biopharmaceutical products, product batches and/or production campaigns. The external device may also comprise reusable hardware that is configured with consumables, which are single-use consumables.

Thus, the comparing of predefined workflow process scheme relating to the selected biopharmaceutical product to at least a part of the tracked operator interactions with the manufacture system and/or to at least a part of the result of the tracked operator interactions may for example be made with regard to connection of hoses for consumables as well as for connections of electrical cables. Further, the comparing may also or instead be made for installed sensors extending into the manufacturing device for sensing characteristics relating to the manufactured biopharmaceutical product and/or the manufacturing device itself.

Thereby, it may be determined that the manufacture system is supplied with the consumables and/or connected to the electrical devices and/or connected to power supply and/or that the sensor(s) are installed in accordance with the predetermined workflow process scheme.

The predetermined workflow process scheme may further comprise characteristics of external devices 120 connected to the manufacture system 110 for the manufacture of the selected biopharmaceutical product. The processing element 150 may then be arranged to obtain information relating to at least one connected external devices, compare the obtained information to characteristics of the connected external device obtained from the predetermined workflow process scheme and determine whether the pre-set criterion set by the predetermined workflow process scheme relating to the characteristics of the external devices connected to the manufacture system is fulfilled.

Thereby, it may be determined that the manufacture system is supplied with the right consumables and/or connected to the right electrical devices and/or connected to right power supply and/or that the right sensor(s) are installed. Further, it may be determined that the external devices of the manufacture system, such as the consumables, are connected in right order and configuration. This increases the safety in that the determination implies that the right product is delivered by the system having the expected properties. By the monitoring system determining right configurations and compliance with operating procedures, and also determining the operator interaction and behaviour in compliance with operating procedures, mistakes by operators, errors in the setup or other deviations may be detected and corrected, and omitted in the first place, which is of great value for verification of a correct production setup prior to qualifying the manufacture system such that the manufacture system subsequently can process fluids and the drug of interest. The above relates also to for example the processing itself and/or tear down of the system.

In different embodiments of the present disclosure the processing element 150 is arranged to detect at least one of the connected external devices 120 in the image and to obtain the information relating to the at least one of the connected external devices based on the detection in the image. The information may be obtained from a bar code, QR code and/or plain text on the connected external device and/or detected characteristics of the connected external device itself, such as shape and/or contours and/or colour and/or appearance (translucent, opaque, reflective etc).

Accordingly, the captured images may be used for detecting the external devices. Thus, as soon as a new external device enters the scene, this may be detected by processing of the images.

Further, any information in the captured images relating to the characteristics of the connected external devices may be used by the system for obtaining information relating to the respective external devices.

The processing element 150 may be arranged to obtain information relating to the at least one connected external device 120 by receiving external device data via wireless or wired communication. In the illustrated example, the manufacture system comprises a receiver 140 arranged to receive wirelessly communicated data. However, such receiver may be located elsewhere within the facility. The operator may even carry an electronic user device having such functionality. The external device data received by the receiver 140 is transmitted to the processing element 150. The external device data relating to the at least one connected external device may comprise identity data. The identity data may be communicated from an RFID tag 122 associated to the external device 120, for example.

Accordingly, the respective external device may be detected by the reception of the external device data such as identity data identifying the individual external device. Thus, as soon as a new external device enters the scene, this may be detected by the reception of the external device data such as the identity data

Further, the received external device data relating to the respective connected external device may be used by the monitoring system for obtaining information relating to the respective external devices.

Further, the determination that the manufacture system is supplied with the right consumables and/or connected to the right electrical devices and/or connected to right power supply and/or that the right sensor(s) are installed, may instead or in addition thereto be made based on the tracking of the operator(s) within the scene. Thus, the plurality of ways of determining that the right external device is connected may provide redundancy in the determination. Thereby, the reliability may be improved using a plurality of sources upon which the determination is based.

The processing element 150 may also or instead be arranged to obtain information relating to the at least one external device 120 by operator input by means of the user interface 160.

The processing element 150 may be arranged to track operator interactions also with at least the at least one external device, compare the tracked operator interactions with the at least one external device to the predefined workflow process scheme relating to the operator interactions with the at least one external device and determine whether at least one pre-set criterion set by the predefined workflow process scheme is fulfilled based on said comparison.

Accordingly, not only operator interactions with the manufacture system and/or the result thereof is tracked but also operator interactions with the external device(s) and/or the result thereof is tracked.

The processing element 150 may be arranged to track the operator when connecting a respective interconnection element to its corresponding external device and/or when installing the respective external device. Thus, the processing element may be arranged to track the operator when connecting to the external device hoses, cables or the like connected to the manufacture system. The processing element may further be arranged to track other procedures for installing the respective external device.

The comparison with the predetermined scheme relating to the selected biopharmaceutical product may then comprise comparing a procedure as determined based on the images with a procedure as indicated by the predetermined workflow process scheme relating to the selected biopharmaceutical product.

The scene captured by the at least one image capture device may at least in part comprise a biopharmaceutical manufacture facility comprising the manufacture system. The processing element 150 may then be arranged to process the images captured by said at least one image capture device to determine at least one of the following:
- before setting up for manufacture of the selected biopharmaceutical product is initiated, determining that the biopharmaceutical manufacturing facility is in a state ready for initiating setting up for manufacture. Thus, the predefined workflow process scheme may comprise a set of criteria which when fulfilled defines that the facility is ready for initiating setting up for manufacture. In a GMP (Good Manufacturing Process) processing environment with a process running in a dedicated production area or room, an essential aspect of determining readiness for setting up the manufacture system is to verify the bill of material (BOM) comprised by the batch protocol. The bill of material typically comprises (single-use) consumables, buffers, vessels, required to configure a manufacture system. The bill of material may also comprise hardware and other components required to execute the process, such as sampling devices, etiquettes etc.

The monitoring system and its processing element 150 may determine that the correct BOM is transferred into the scene by verifying identity, shelf life validity etc. In case of any variability detected in the BOM against the batch protocol, the monitoring system may determine whether alternative components, for example connector or fluid bag variants, are compatible with the pre-determined batch protocol or variations of a pre-determined batch protocol. The scene may be defined as the complete operating area, such as a room, facility or area. However, for certain operations or events, the monitoring system and its processing element 150 may focus on a smaller area of said scene, for example when guiding and monitoring operator interactions at a specific operating step, specific equipment and/or specific parts comprised by the BOM. For example, the connection of a specific tubing to an inlet connection of a chromatography system may focus on sensing, monitoring and documenting solely equipment, parts and items of the BOM and operator(s) specifically relevant for establishing said connection between tubing and system inlet.

The monitoring system may also provide assistance and guidance in updating or modifying a batch protocol and achieving approvals or provide guidance to efficient operator or QA initiated approvals of modified batch protocols or new revisions of batch protocols. The system may conduct or provide assistance in a risk analysis for said revisions.
- whether external devices present in the facility have been moved or exchanged during setting up for manufacture/processing or during manufacture/processing. If it is detected that external devices have been moved or exchanged during setting up for manufacture or during manufacture in a way not in line with the predefined workflow process scheme and/or in a way which could compromise the quality of the manufactured biopharmaceutical product it may be determined that at least one of the pre-set criteria is not fulfilled.
- operator movements within the facility during setting up for manufacture or during manufacture. If it is detected that an operator during setting up for manufacture or during manufacture has been moving around in the facility or has been interacting with the manufacture system in a way or at instances not in line with the predefined workflow process and/or in a way which could compromise the quality of the manufactured biopharmaceutical product it may be determined that at least one of the pre-set criteria is not fulfilled. For example, it may be detected that unauthorised personnel have been in the facility during setting-up for manufacture or during manufacture.

The processing element 150 may further be arranged to provide an indication that the process is verified for those operator interactions and/or for the result of those operator interactions determined to fulfil the at least one pre-set criterion set by the workflow process scheme.

Thus, the processing element 150 is in accordance with this example arranged to first determine that the process fulfils pre-set criteria set by the workflow process scheme and then to make a verification verifying the determination that the process fulfils pre-set criteria set by the workflow process scheme.

This procedure characteristically requires redundancy. Thus, for example, if the determination is made using first source(s), the verification is made using other source(s). It may be that some of the pre-set criteria can be validated while for other pre-set criteria redundancy is lacking and thus no verification can be made. In a conventional prior art system, a first operator may complete an operations step, for example the connection of two fluid lines, and confirm this action with a signature in the batch record. For important or critical actions, a second operator is verifying the correctness and completion of the operating step as well as for the documentation provided by the first operator through inspection and provision of a second signature in the batch record. In the monitoring system proposed herein, both first and second operator signatures may be omitted provided that the system is able to obtain sufficient information on correctness, completion and documentation of the step by its image analysis and sensing features, which may comprise machine vision and machine inspection technology. Any intermediate state, where one or both of the two operators are in total or partly relieved of any identification, documentation or verification action will provide improvements in accuracy, speed and quality in setting up or operating the manufacture system. In using this verification procedure, automatic / computer aided verification is provided. This increases the safety in that the verification implies that the right product is delivered by the system having the expected properties.

In Fig 2, an example of a user interface 260 suitable for a monitoring system as discussed in relation to figure 1 is illustrated.

The user interface 260 comprises a user input element 261. The user input element 261 may for example comprise a keyboard, a touchscreen or the like. The user input element 261 may be arranged to receive operator input for selecting a biopharmaceutical product for manufacture. The user input element 261 may be arranged to receive operator input relating to at least one external device, as discussed in relation to figure 1. The user input element 261 may be arranged to receive operator input relating to other information about the setting up for manufacture and/or the manufacture set-up itself, which information may be used in the comparison with the predefined workflow process scheme relating to the selected biopharmaceutical product.

The user interface 260 comprises further in the illustrated example at least one display element 162. The at least one display element is arranged to display instructions for setting up for manufacture of the selected biopharmaceutical product, the status of operations and/or conditions of components or the system, and/or projections, predictions or indications for future conditions of the system or its components. The processing element 150 as discussed in relation to fig 1 may then be arranged to feed instructions to the display relating to a next or current step and/or to feed to the operator feed-back information relating to the carrying out of a preceding step based on the tracking of the operator in the images captured by the at least one image capturing device.

The at least one display element 162 may comprise an augmented reality display for example formed in glasses worn by the operator. Other technologies may be used instead or in addition thereto, such as light guide systems and/or projections, haptic feedback, audio etc.

In another example the at least one display element 162 may comprise an electronic ink (e-ink) display. The information shown on E-ink displays may be altered in dependence of user interaction steps, process status etc. with the advantage that very little or no further energy has to be provided to the readable display in between events of altering information. In other examples, display elements, and/or also sensors may be powered to energy harvesting devices. Energy harvesting technologies, such as for example recovery of electromagnetic radiation or solar panels harvesting indoor light, may be utilized to eliminate the need for batteries at wirelessly connected devices.

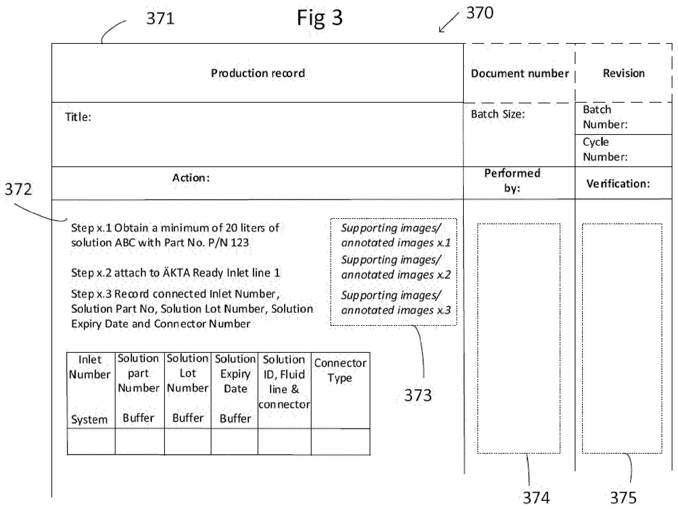

Figure 3 illustrates schematically contents stored in a memory 370 of a monitoring system for manufacture of a plurality of biopharmaceutical products for example as discussed in relation to figures 1 and 2. The memory 370 stores permanently or temporarily workflow process schemes associated to the respective biopharmaceutical product. The predetermined workflow process scheme associated to each product may comprise at least one of the following:
- data identifying which steps are to be carried out for setting up for manufacture
- data identifying an order and/or timing for carrying out the steps for setting up for manufacture, for those steps this is relevant
- data identifying a procedure for carrying out each step and
- data identifying which external devices are to be connected to the manufacture system, data identifying a configuration of interconnections between the manufacture system and external devices,
- data identifying characteristics of the manufacturing facility in a state ready for initiating setting up for manufacture,
- data identifying allowed external devices present in the facility and/or allowed movements of devices within the facility and/or allowed exchanges of devices within the facility,
- data identifying allowed operator movements within the facility, such as number of operators allowed or required in the facility and/or identity of authorised operators allowed in the facility and/or timings when operators are allowed to be in the facility and/or areas allowed for operators in the facility, wherein the allowed area may be associated to an authorisation level of the individual operator, etc.

In the example of Figure 3, a production record 371 relating to a biopharmaceutical product is illustrated, and Figure 3 is showing an excerpt of such a record for a specific step of connecting a fluid line to an inlet of a flow path in a chromatography system. The production record 371 comprises recorded data relating to said predetermined workflow process scheme. The production record 371 may be comprised in the predetermined workflow process scheme 372 relating to a selected biopharmaceutical product.

In the illustrated example, the predetermined workflow process scheme defines a plurality of activity steps x.1, x.2, x.3. The illustrated activity steps exemplify the connection of a buffer vessel and corresponding fluid line to an inlet of a chromatography system

The first step x.1 defines that a predetermined solution having a predetermined part number should be obtained. The production record 371 comprises in the illustrated example an area 373 for storage of supporting images or video films showing the execution of the first step or the result thereof. The supporting images or video films may be annotated or not.

The production record 371 comprises further an area 374 for recording a signature that the step has been executed. The signature may be provided by the operator having performed the step or another person. The signature may instead be computer generated based on the comparison between captured images and the predetermined workflow process scheme, optionally in combination with other information obtained from additional sensor elements.

The production record 371 comprises further an area 375 for recording a verification that the first step has been successfully performed. The verification may be provided another person that the person who provided the signature that the step was executed. The verification may instead be computer generated based on the comparison between captured images and the predetermined workflow process scheme, optionally in combination with other information obtained from additional sensor elements.

The same applied for the subsequent steps:
- supporting images/video(s), and/or information from additional sensor elements, annotated or not may be stored in the area 373,
- a signature, electronic or manual, may be provided in the area 374 for a signature indicating that the step has been executed
- a verification, manual or electronic may be provided in the area 375 for verification that the step has been successfully performed.

Further, the production record 371 comprises records of connections and external devices and characteristics thereof in accordance with the predetermined workflow process scheme. In the illustrated example the records comprise
- Inlet number-system
- Solution part number- Buffer
- Solution Lot Number- Buffer
- Solution Expiry Date - Buffer
- Solution ID, Fluid Line & Connector
- Connector type

Further, for each record there may be a signature and a verification, as described above. Further, for each record there may be supporting images/video and or other information obtained from additional sensor elements, annotated or not, as described above.

Thus, the illustrated production record 371 comprises data indicating which tracked operator interactions and/or results thereof fulfil the pre-set at least one criterion set by the workflow process scheme and/or verification data indicating which processes are verified.

As is described above the illustrated production record comprises also images/video and or other information obtained from additional sensor elements, annotated or not upon which the data indicating which tracked operator interactions or results thereof fulfil the pre-set at least one criterion set by the workflow process scheme at least partly is based and/or upon which the verification data at least partly is based.

The proposed monitoring system obtains data, which may be used in determining whether the at least one pre-set criterion set by the workflow process scheme is fulfilled and possibly for verification. The obtained data may be stored.

The verifications made and amount of data stored are characteristically at least at a minimum level requirements defined by the manufacturer, authorities and /or regulating bodies, such as FDA.

The obtained data may further be stored for use in trouble shooting, for data and workflow analysis, and for improving insights and models on cause-effect relationships, such for example by applying mechanistic or statistical modelling and data analysis across the whole or parts of the manufacturing process including manufacturing setup and BOM.

The proposed monitoring system allows for computer-aided or automated verification of the setting up of the manufacture system for the selected biopharmaceutical product.

The computer aided/automated verification leads to minimizing down time when setting up the manufacture system for manufacture of a new biopharmaceutical product.

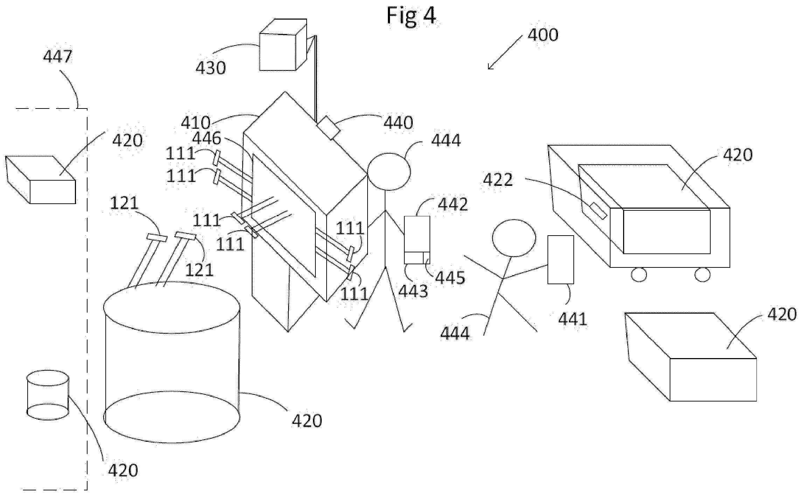

Fig 4 discloses a scene 400 where operators are setting up for manufacture of a selected biopharmaceutical product. The setting up for manufacture and the following manufacture of the biopharmaceutical product may be supported by the monitoring system for example as disclosed in any of the figures 1, 2 and 3.

The manufacture of the biopharmaceutical products is characteristically conducted under current good manufacturing practice, cGMP. The manufacture involves characteristically one or multiple unit operations for upstream production (i.e. bioreactor) and/or downstream product purification. The manufacture relies characteristically as much as possible on single-use technologies across the manufacturing process. This means that disposable consumables, hoses etc. are used. Thereby a facility and equipment used for the manufacture of the biopharmaceutical product can easily be cleaned before initiating setting up for manufacture of a new biopharmaceutical product, for example by disposal and replacement of used consumables that have been in product contact during a completed process.

An image capturing device 430 in the form of a camera is arranged to capture the scene 400. The scene 400 may cover the manufacture facility for the manufacture of the biopharmaceutical product. The manufacture facility may be a secluded space in such a room. The image capturing device may be arranged to capture an entrance to the scene. Typically, an entrance to the scene may be a transfer and/or inspection area 447 for incoming material, for example material according to the BOM.

BOM verification may be performed in the transfer and/or inspection area 447 adjacent to the clean production and processing zone. The BOM verification may be performed on a desk, bench or a shelf where incoming material of the BOM is verified. The image capturing device and/or other sensor elements may be used for the verification to 'check in material' prior to installation.

A manufacture system comprising at least one manufacture device 410 is arranged in the scene 400.

A plurality of external devices 420 are arranged within the scene. The external devices 420 may comprise external devices comprising a flow kit 446 and consumables mounted to a manufacture device such as a chromatography system and/or a bioreactor. The external devices may comprise a buffer bin and/or a vessel connectable to the manufacture system.

In the illustrated example, the flow kit 446 is mounted at the front of the manufacture device 410 in the form of a chromatography system, for example an ÄKTA ready system (GE Healthcare). The flow kit 446 has first connection elements 111. In the illustrated example, the first connection elements 111 comprises fluid inlets, fluid outlets and connections 111 to an external device 420 in the form of a chromatography column, for example a Ready To Process column (GE Healthcare). Connection elements 111 may be provided in the form of aseptic connectors, for example ReadyMate connectors (GE Healthcare).

At least some of the first connector elements 111 of the manufacture device 410 and at least one second connector element 121 of at least one external device 420 are connectable to each other. The connector elements 111, 121 may be intended to be connected directly to each other by interconnections/interconnection elements such as hoses, cables or the like. In the illustrated example, the second connector elements 121 of the external device 420 in the form of a column is intended to be connected to the corresponding first connector elements 111 of the manufacture device 410.

The connector elements 111 may be adapted for connection to external devices 420 such as consumables. The connector elements 111 may be adapted for electrical communication and may for example form electrical contacts.

In the illustrated example, the manufacture device 410 comprises a receiver 440 arranged to receive wirelessly communicated data relating to at least one of the external device(s), the manufacture device 410, a reader and other equipment within the scene. However, such receiver 440 may instead or in addition thereto be located elsewhere within the facility and provide data to the monitoring system. In the illustrated example, an operator carries an electronic user device 441 having such receiver 440. The wirelessly communicated data may for example be communicated from RFID tags 422 or other transmitter elements 445 associated with the external device(s) and/or the manufacture device 410 and/or other equipment and/or material within the scene, such as connectors, fluid lines, fluid bags, flow kits, columns, filters etc. The RFID tag or other transmitter elements may be arranged for short range communication. In one example, communication is characteristically obtained when the transmitter and receiver are in the same room. Communication may be obtained by Bluetooth, WiFi, RFID, NFC etc.

For example, an external device may comprise a memory or have access to a memory comprising at least some of the following data:
- part number
- Lot Number
- Expiry Date
- ID
- Characteristics of external device (such as type, unit operation(s), consumable, probe etc), further details about the constitution/function of the external device, size, etc)
- Connector type
- State/status of connections (packed, unwrapped and prepared for connection "ready to connect", faulty or correct state prior to connection, faulty or correct state in established connection)
- Suitable interconnecting elements for connecting the external device (clamp type etc)
- Sensor type(s) associated to the external device
- Environmental data such as temperatures the external device has been subjected to

At least a part of the data above may be communicated to the receiver 440 upon request and/or when communication is established and/or a sensor signal is obtained by any sensor associated to the external device. The receiver 440 may be a wireless receiver or sensor forming a complement to traditional wired sensors and wire transmitted data.

For example, the manufacture device may comprise a memory or have access to a memory comprising the following data:
- part number
- Lot Number
- Connector type(s)
- ID
- Characteristics of the manufacture device (such as type (chromatograph, bioreactor), further details about the constitution/function of the manufacture device, size, etc)

At least a part of the data above may be communicated to the receiver 440 upon request and/or when communication is established.

The other equipment may comprise the interconnecting elements for connection of the manufacture device to the external devices. The interconnecting elements may include hoses, cables, clamps or the like. The other equipment may comprise a memory or have access to a memory comprising the following data:
- ID
- Characteristics of the equipment (such as Equipment type (electrical cable, hose clamp etc), further details about the constitution/function of the equipment, size, etc)
- Sensor type(s)associated to the equipment

At least a part of the data above may be communicated to the receiver 440 upon request and/or when communication is established and/or a sensor signal is obtained by any sensor associated to the equipment above device.

Accordingly, the respective external device and/or other equipment may be detected by the reception of the external device data such as identity data identifying the individual external device. Thus, as soon as a new external device and/or other external device enters the scene, this may be detected by the reception of the external device/other equipment data such as the identity data.

Further, a status change sensed by a sensor of the respective external device and/or other equipment may be detected by the reception of the external device/other equipment data including said sensor status data. Thus, as soon as a status change of a sensor of an external device and/or other equipment is sensed, this may be detected by the reception of the external device/other equipment data.

Further, the received external device data/other equipment data relating to the respective connected external device may be used by the monitoring system for obtaining information relating to the respective external devices/other equipment.

Further, labels or tags, such as RFID tags 422, associated with the external device(s) and/or the manufacture device 410 and/or other equipment and/or material within the scene, and information contained by said labels or tags may also be altered and updated to reflect changes in status, condition or use of said components. Information may be written to the tags to update the contents thereof.

In the illustrated example of figure one of the operators 444 holds other equipment 442 for example in the form of a clamp, such as a tube clamp (tubing not shown). The other equipment is associated to a sensor 443 and transmitter element 445 associated to a memory (not shown).

Further, operating personnel within the scene may also be tracked by means of the camera. Thereby, it may be registered with which external devices and/or other external equipment connected to the manufacture device or not the operators interact.

Thus, operators entering the facility and acting within the scene are tracked and their interactions with the devices/other equipment of the facility may be tracked.

Information redundancy may be used to verify that detected events have taken place. For example, if a sensor detects a status change, the images from the image capturing device may be used for verifying that at the timing and/or position of the status change of the sensor, the position of the operator in the images at that timing may be used for verifying that an operator performed an activity on the equipment/external device to which the sensor is associated. Further, the images may be processed to track the operator manoeuvres in detail to ensure that the operator connected the equipment/external device in a correct manner and/or in accordance with pre-determined requirements for order and/or timing. In addition thereto or instead received information relating to equipment/external devices may be used for ensuring that that correct equipment/external devices were involved in the connecting activity. In addition thereto or instead image processing may be used for ensuring that correct equipment/external devices were involved in the connecting activity. Image processing may be applied at various levels of detail and sophistication including methods of machine vision and machine inspection, which may comprise recognition of written text and information on labels, bar and OCR code information etc. to relief the operator from reading, processing and/or documenting this information, for example in the batch record.

In another example, when image processing detects that a new external device and/or other equipment has entered the facility, data received by the receiver 440 may be used for confirming this. Further, the images may be processed to track the operator manoeuvres in detail to ensure that the operator brought the other equipment/external device to the facility at the timing of the reception of data via the receiver 440. Further or in addition thereto, image processing may be used to confirm that the characteristics of the other equipment/external device coincides with the characteristics of the other equipment/external device as determined by the data received by the receiver 440.

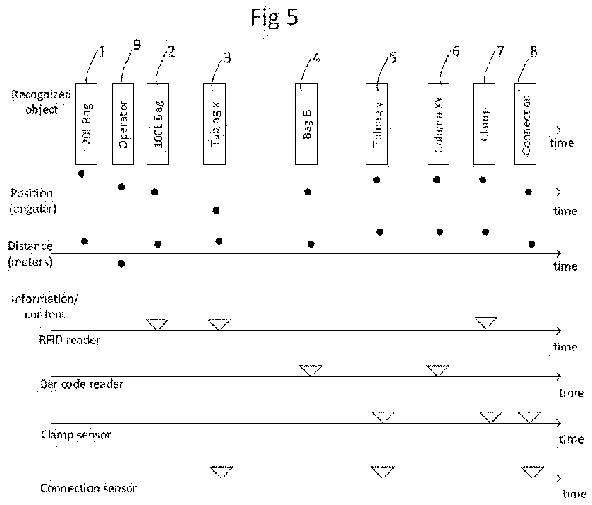

Figure 5 discloses a scheme illustrating a time line for recognition of external devices including consumables and reusable hardware, and operators and other equipment within a scene. The time line for recognition allows to detect, inspect, monitor, verify and document equipment/external devices/ as well as operators, their interaction with equipment/external devices and the results of this interaction

The external devices of this illustrative example comprise consumables for the manufacture device. The other equipment comprises in the illustrated example clamps, which here feature a wireless detection of clamp status representing an open or closed valve and fluid line, the latter when the operator closes the clamp and the clamp pinches the fluid line.

In the illustrated example, a 20L bag 1 consumable is recognized at a timing along the time line. The timing of the recognition may be recorded. In the illustrated example, a position of the recognised 20L Bag 1 is recorded. In the illustrated example, the position comprises a recoded angular position of the 20L Bag 1. The angular position may be given in relation to a predetermined reference direction. The position comprises in the illustrated example further a distance. The distance may be given in relation to a predetermined reference point. The angular position and the distance may be determined based on the images captured by the image capture device.

Angular positions and distances from the image capture device (camera) may be translated to and represented by Cartesian 2 and 3 dimensional coordinates and the scene may be re-constructed and displayed in mixed reality involving virtual and or augmented reality displays.

Further, the characteristics of the 20L Bag object may be identified by processing of the captured images to determine that the identified object is a 20L Bag 1.

Further, in the illustrated example, an operator 9 is recognized at a timing along the time line. The timing of the recognition may be recorded. In the illustrated example, a position of the recognised operator is recorded. In the illustrated example, the position comprises a recoded angular position of the operator 9. The angular position may be given in relation to a predetermined reference direction. The position comprises in the illustrated example further a distance. The distance may be given in relation to a predetermined reference point. The angular position and the distance may be determined based on the images captured by the image capture device.

Further, in the illustrated example, a 100L bag 2 is recognized at next timing along the time line. The timing of the recognition may be recorded. In the illustrated example, the position of the recognised 100L Bag 2 is recorded. In the illustrated example, as discussed above, the position is recorded as an angular position of the object. The position comprises in the illustrated example further a distance. The angular position and the distance may be determined based on the images captured by the image capture device.

Beyond the representation of data shown in Figure 5, any information obtained on parameters including position, status and actions of operators, external devices, other equipment and other objects in the scene, with resolution in space and time, may of course be presented, evaluated and documented in various and different formats and graphical presentations.

Further, the characteristics of the 100L Bag 2 object may be identified by processing of the captured images to determine that the identified object is the 100L Bag.

Further, at the timing of recognizing the 100L Bag 2, the RFID reader reads an RFID tag associated to the 100L Bag. Thereby, redundancy and/or other information such as an identity of the 100L Bag may be obtained.

The reading of the RFID tag may be associated with an operator interaction, for example, the activation of the reader, and the reading may be associated with tracked operator and/or reader position in time and space.

Further, at yet another timing a Tubing x 3 is recognized. The timing of the recognition may be recorded. In the illustrated example a position of the recognised Tubing x 3 is recoded. In the illustrated example, as discussed above, the position may be is recorded as an angular position and a distance using the images captured by the camera. Further, at the timing of recognizing the Tubing x 3 a RFID reader reads an RFID tag associated to the Tubing x 3. Thereby, redundancy and/or other information such as an identity of the Tubing x 3 may be obtained. Further, at the timing of recognizing the Tubing x 3, a connection sensor senses that the tubing x 3 has been connected. The connection sensor may be a sensor component or sensor components attached to one or two connectors involved in the connection that is established, providing wired or wireless sensor feedback. However, the connection sensor may be provided by other sensing means, for example image analysis and machine vision capability that may recognize the identity of at least one of the connectors and may recognize the successful conduction and completion of a connection process. Thus, additional information, which may provide redundancy, relating to the set-up of the manufacture system is obtained.

At yet another timing a Bag B 4 is recognized. The timing of the recognition may be recorded. In the illustrated example, a position of the recognised Bag B 4 is recorded. In the illustrated example, as discussed above, the position may be recorded as an angular position and a distance using the images captured by the camera. Further, at the timing of recognizing the Bag B 4, a bar code reader reads a bar code. The bar code reader may for example be carried by the operator. Alternatively, the barcode is identifiable from the captured images. The barcode may be present at the tubing itself or packaging thereof.

Thereafter, at a next timing, a Tubing y 5 is recognized. The timing of the recognition may be recorded. In the illustrated example, a position of the recognised Bag y 5 is recorded. In the illustrated example, as discussed above, the position may be recorded as an angular position and a distance using the images captured by the camera. Further, at the timing of recognizing the Tubing y 5, a connection sensor senses that the tubing y 5 has been connected. Thus, additional information, which may provide redundancy, relating to the set-up of the manufacture system is obtained.

Further, a clamp sensor status change is detected at the tubing y 5. The clamp sensor detects whether a tubing clamp, characteristically a manual pinch valve, is in an open position or a closed position. Information relating to the position of the operator may be used provide further information that the operator was at the location of the tubing clamp when the clamp sensor status change was detected.

Thereafter, at yet another timing, a column XY 6 is recognized. The timing of the recognition may be recorded. In the illustrated example, a position of the column XY 6 is recorded. In the illustrated example, as discussed above, the position may be registered as an angular position and a distance using the images captured by the camera. Further, at the timing of recognizing the column XY 6, a bar code reader reads a bar code associated to the column XY 6. Alternatively, the barcode is identifiable from the captured images. The barcode may be present at the column XY 6 itself.

At a next timing, a clamp 7 is recognized. The timing of the recognition may be recorded. In the illustrated example, a position of the recognised clamp 7 is recorded. In the illustrated example, as discussed above, the position may be recorded as an angular position and a distance using the images captured by the camera.

In practice, the clamp 7 may be recognized at the timing of activation of a clamp activation signal from a clamp sensor associated to the clamp 7. The clamp senor activation signal may be transmitted upon detection that the clamp is connected. The tracking of the operator may then be used for identifying the clamp 7 in the images for use in determining the position of the clamp 7. The operator is characteristically at the location of the clamp 7 at the timing of the clamp sensor activation signal. Thus, the images captured at the timing of the activation of the clamp sensor signals are characteristically used for determining the position of the clamp 7 manoeuvred by the operator.

Alternatively, or in addition thereto, the clamp 7 may be recognized in a open or closed state by information obtained by the image capture device and evaluated by processing element.

Further, at the timing of recognizing the clamp 7, an RFID reader reads an RFID tag associated to the clamp 7. Thereby, redundancy and/or other information such as an identity of the clamp 7 may be obtained.

At a next timing, a tube connection 8 is recognized. The timing of the recognition may be recorded. In the illustrated example, a position of the recognised tube connection 8 is recorded. In the illustrated example, as discussed above, the position may be recorded as an angular position and a distance using the images captured by the camera.

In practice, the tube connection 8 may be recognized at the timing of activation of a clamp activation signal from a clamp sensor associated to the tube connection 8. The clamp senor activation signal may be transmitted upon detection that the clamp is connected. The tracking of the operator may then be used for identifying tube connection 8 in the images for use in determining the position of the tube connection 8. The operator is characteristically at the location of the tube connection 8 at the timing of the clamp sensor activation signal. Thus, the images captured at the timing of the activation of the clamp sensor signals are characteristically used for determining the position of the tube connection 8 manoeuvred by the operator.

Further or instead, the tube connection 8 may be recognized at the timing of activation of a tube connection signal from a sensor of the tube connection 8. The tube connection sensor signal may be transmitted upon detection of connection. The tracking of the operator may then be used for identifying tube connection 8 in the images for use in determining the position of the tube connection 8. The operator is characteristically at the location of the tube connection 8 at the timing of the tube connection sensor signal. Thus, the images captured at the timing of the detection of the tube connection sensor signals are characteristically used for determining the position of the tube connection 8 manoeuvred by the operator.

The monitoring system may keep track, control and advise the user regarding the optimal physical positioning and orientation of objects and components within the manufacturing space and scene 400. For example, the monitoring system may propose a floor plan for positioning of components and instruct the user to arrange said components according to the proposed floor plan. The floor plan may change throughout a process for optimal utilization of space within the clean room and/or for optimal execution of the manufacturing process. The monitoring system may further comprise a machine learning and optimization component such that the floor plan is further optimized, for example by analysing process performance, user interaction and space utilization from previous batches.

The monitoring system may further allow for integration of robotics into the workflow and it may optimize the interaction of robotics and the user within the manufacturing system, process, facility and/or the overall workflow. For example, automatic self driving material movers can be controlled in concert with manual user interaction, the integration and coordination of all resources may be optimized and improved by machine learning and Al based on analytics.

The tubing and bags are characteristically single use and disposable products. The scheme as set out in figure 5 or a similar scheme may for example be applicable in setting up of a chromatography system with a buffer bag on the inlet side and another buffer bag on an outlet side.

The scheme illustrating a time line for recognition of external devices and other equipment within a scene as discussed above in relation to figure 5 may be used for determining whether at least one pre-set criterion set by the workflow process scheme in accordance with this disclosure is fulfilled. The scheme illustrating a time line for recognition of external devices and other equipment within a scene as discussed above in relation to figure 5 may also be used for verification in accordance with this disclosure. The scheme illustrating a time line for recognition of external devices and other equipment within a scene as discussed above in relation to figure 5 may also be used for verification in accordance with this disclosure. The scheme illustrating a time line for recognition of external devices and other equipment within a scene as discussed above in relation to figure 5 may also be used for improvement of manufacture processes in accordance with this disclosure. Process improvement and/or optimization may be for example be provided through 'time lapse' features that compress, condense or filter events according to one or multiple objectives, which may differ depending on the type of analysis. For example, one time lapse feature could focus solely on the display, review, and/or analysis of user interactions with a certain component. These specific interactions may then be scrutinized to improve efficiency, safety or robustness of a process.

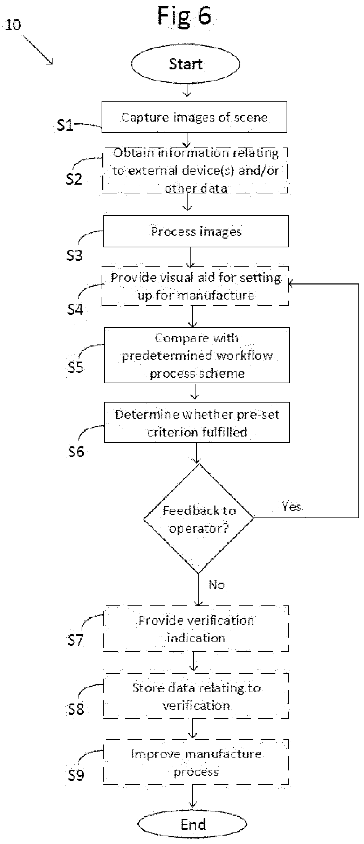

In figure 6, a method 10 for monitoring a set-up for manufacture and/or setting up for manufacture and/or after tearing down after manufacture of a selected biopharmaceutical product, is illustrated.

The method comprises a step of capturing S1 images of a scene comprising a manufacture system. The manufacture system has capability to manufacture a plurality of biopharmaceutical products. The manufacture system may have a plurality of first connector elements for connection to external devices.

The method further comprises a step of processing said captured images S3 to track operator interactions and/or a result of operator interactions within the scene. The method further comprises a step of comparing S5 at least a part of tracked operator interactions and/or at least a part of the result of the tracked operator interactions with the manufacture device to a predefined workflow process scheme relating to the selected biopharmaceutical product. The method further comprises a step of determining S6 whether at least one pre-set criterion set by the workflow process scheme is fulfilled based on the comparison.

The method may also comprise a step S2 of also obtaining information relating at least one connected or connectable device or other equipment. At least a part of the obtained information may then be used in the comparison S5 to characteristics of the connected external device obtained from the predetermined workflow process scheme and the determination S5 whether the pre-set criterion set by the predetermined workflow process scheme relating to the characteristics of the external devices connected to the manufacture device is fulfilled.

The method may further comprise a step of providing S4 visual, haptic and/or audio aid for setting up for manufacture. The visual aid may for example be displayed on a screen or presented as an augmented reality presentation for example formed within glasses worn by the operator. The visual/oral aid provided may be based on tracking of the operator and possibly the predefined workflow process scheme to identify which activity the operator is involved with. Visual/oral support is then provided based on the identified activity. The data for support is for example stored in association with each activity of the predetermined workflow process scheme.

Feed-back to the operator may be provided related to the performed activity. The operator may then have the chance to re-do the activity or at least learn for the next time. The level of operator guidance and feedback may then be situationally adjusted depending on the operator's competence level, experience and request for a certain level and detail in guidance.

Thus, as is understood from the above the determination whether pre-set criteria are fulfilled may be made continuously as the operator(s) work on setting up of for manufacture of the selected biopharmaceutical product. Alternatively, the determination is made once the setting up for manufacture is finished.

To sum up, the method for monitoring a set-up for manufacture and/or setting up for manufacture of a selected one among a plurality of biopharmaceutical products determines which pre-set criteria set by the workflow process scheme are fulfilled and which are not, or at least it cannot be determined whether they are fulfilled. For those potential pre-set criteria which are not fulfilled or at least where it cannot be determined whether they are fulfilled, a report may be provided by the system. Those criteria may be checked manually.

It may be advantageous to include a measure of the accuracy and thus reliability in the determination provided by the processing element 150 with regard to whether the respective pre-set criterion is fulfilled. Based on the accuracy and reliability in the determination, the system may autonomously decide that a pre-set criterion is fulfilled when the accuracy and reliability in this decision is high. In other cases, when information is not sufficient to allow an autonomous decision and reliability is determined as not sufficient, additional operator input may be requested. In some instances, the system 150 may learn from repetitive execution or from an additional data and knowledge base, for example improving image data in of the memory, such that accuracy is improved over subsequent processes resulting in more autonomous decisions being taken by the systems and/or less additional user input required. For example, if the predetermined workflow process scheme indicates that a particular valve should be open, the reliability measure could for example indicate either that the valve is open, that the valve is closed or that the status of the valve is undefined. Thus, the reliability measure could indicate CORRECT, FAULT or UNDEFINED. Alternatively, the reliability measure could comprise a percentage. The reliability measure may be based on the quality in the available data, the number and/or types of sources used for the determination of whether the pre-set criterion is fulfilled, etc.

Further, where it has been determined that the pre-set criteria has been fulfilled, a verification indication may be provided S7. The verification indication is preferably obtained using other information that the information used for the determination that the pre-set criteria is fulfilled. Data relating to the verification may then be stored S7. The verification is characteristically made once the setting up for manufacture is finished.

It may be advantageous to add a measure of the reliability to the verification in the same or similar manner as discussed above in relation to the in the determination of whether the respective pre-set criterion is fulfilled.

Data relating to the verification may be stored S8. Thus, the information upon which the verification is made may be stored.

Data relating to the determinations whether the pre-set criteria set by the workflow process scheme are fulfilled may be stored.

Other data may also be stored obtained during setting up for manufacture.

Also, any amendments made within the scene for example made during production may also be stored. The amendments may include that external devices and/or other equipment have been moved or compromised with. This may for example be useful in possible subsequent troubleshooting.

After installation is verified and approved, the processing of the biological product is undertaken, which is typically some kind of fluid processing (bioreactor, chromatography, filtration). Here, many operator interactions are required, like sampling of fluid, actuation of tubing clamps etc.

Teardown of the manufacture system after processing is also a step where the operator has to follow a protocol that may be provided by the monitoring system. Interactions and results are monitored, verified and documented also in this step.

The stored data may be used in a subsequent step manufacture process is improved S9. Thereby, the production of the final biopharmaceutical product may be refined and the potential deviations in the final biopharmaceutical product may be decreased for each time the biopharmaceutical product is manufactured.

In detail, the visual/audio aid may be improved based on (statistical) analysis of previous operator activities for setting up for manufacture and the characteristics of the final biopharmaceutical product thereby manufactured.

The decision making made by the system may also be improved by letting the operator in this improvement step go through UNDEFINED results in determination whether the predetermined pre-set criteria are fulfilled and/or in verification and to indicate whether the pre-set criteria were fulfilled or not and whether a verification can be made or not in the cases indicated as UNDEFINED by the system.

Machine learning may be used in this improvement step S9. The learning may be used for refining one or a plurality of steps in the process, such as steps S2, S3 and S6.

The method may be implemented in a computer program product comprising at least one non-transitory computer readable storage medium having computer-executable program code instructions stored therein, wherein the computer-executable program code instructions comprise program code instructions configured, when executed perform the method.

Learnings and system intelligence may be used for refining, updating and improving the batch protocol for example during 'technical runs' by recording and documenting parts of the batch protocol. Further, information obtained (i.e. mixed reality output and batch protocol) may be used for operator training purposes, troubleshooting, data analysis etc.

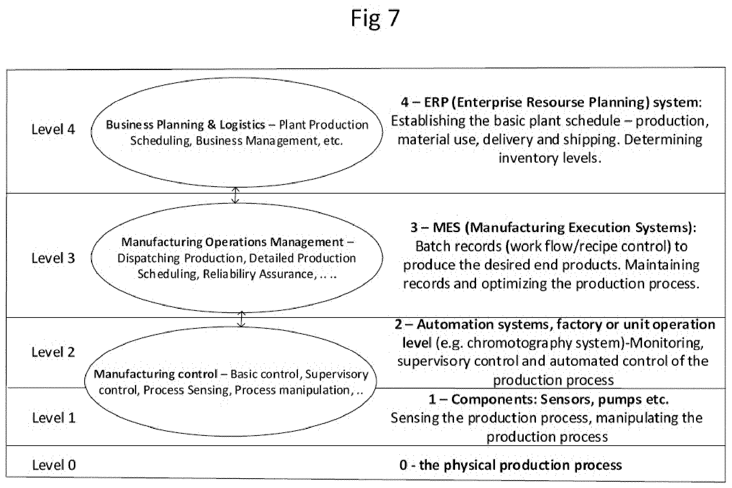

Figure 7 illustrates a scheme illustrating different levels of providing manufacture support aligned with the ISA95 standard. A level 0 illustrates a physical production process. In level 1, the components, such as sensors, pumps etc. of the physical production process are defined. The physical process is sensed by means of sensors and the production process may be manipulated. Level 2 relates to automation systems. Level 2 may be defined as a factory or unit operation level. In level 2, monitoring and supervisory control and automated control of the process is obtained. By implementing levels 1 and 2, manufacturing control is achieved. The manufacturing control involves basic control, supervisory control, process sensing and process manipulation.

Level 3 is a Manufacturing Execution System, MES, level. In the illustrated example, at level 3, batch records are controlled to produce desired end products. The batch records are intended for work flow and/or recipe control. The batch records may be maintained and/or the production process may be optimized. The use of the predetermined workflow process scheme as disclosed herein allows for manufacture support primarily at this level, namely the control, execution and documentation of batch records. To take full advantage of the monitoring system as described herein, batch records are preferably managed electronically and workflow instruction as well as input and feedback to the electronic batch record (eBR), such as by sensors, are transferred electronically. This level 3 may be denoted a level of manufacturing operational management. This level covers dispatching production and/or detailed production scheduling and/or reliability assurance.

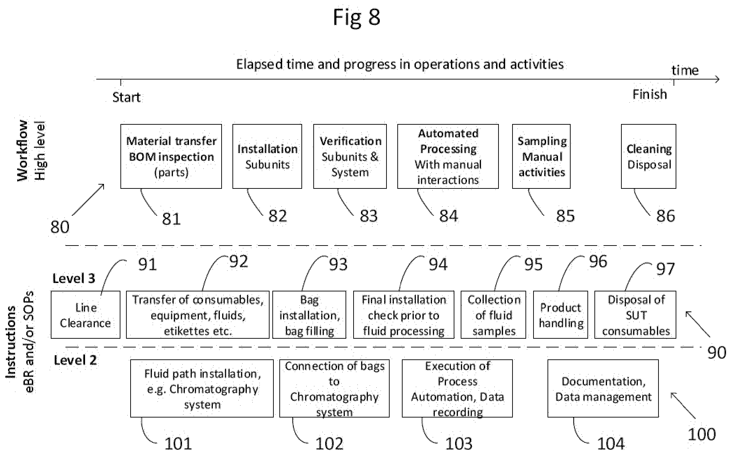

However, at least parts of the workflows may be defined at level 2. This will be more discussed in relation to figure 8.

Level 4 is an Enterprise Resource Planning, ERP, level. Level 4 is a level for business planning and logistics. This level involves plant production scheduling and business management.

Fig 8 illustrates an example of a scheme for manufacture of a predetermined biopharmaceutical product.

The scheme comprises in the illustrated example a high level workflow 80 for the manufacture of the predetermined biopharmaceutical product. In the illustrated example, the high level work flow 80 starts with material transfer and/or BOM inspection 81. Thereafter, an installation step for installation 83 of the manufacture system is performed. Thereafter, the installation is verified 83. Thereupon, automated processing 84 possibly with manual interactions is performed. Thereafter, a sampling step 85 is performed for sampling manual activities. Thereafter the scene of the manufacture is cleaned 86. In this step, single use products are disposed. Steps may be added and/or removed from this high level work flow 80.

Further the scheme may comprise instructions 90, 100 for the manufacture of the predetermined biopharmaceutical product. The instructions comprise for example Standard Operation Procedures, SOPs, and/or an electronic batch record, eBR. The instructions may belong to either level 2 or level 3 or a combination thereof in the different levels of providing manufacture support aligned with the ISA95 standard (by ISA, International Society of Automation), as discussed in relation to figure 7.

In the illustrated example Level 3 instructions 90 comprise instructions for Line clearance 91. This instruction characteristically precedes or may be considered an initialization of the material transfer and/or BOM inspection 81.

In the illustrated example, the Level 3 instructions comprise further instructions 92 for transfer of consumables, and/or equipment and/or fluids and/or etiquettes.

This instruction characteristically belongs to material transfer and/or BOM inspection 81 and/or installation 82.

In the illustrated example, the Level 3 instructions comprise further instructions 93 for bag installation and/or bag filling. This instruction characteristically belongs to installation 82 and/or verification 83.

In the illustrated example, the Level 3 instructions comprise further instructions 94 for final installation prior to fluid processing. This instruction characteristically belongs to verification 83 and/or automated processing 84.

In the illustrated example, the Level 3 instructions comprise further instructions 95 for collection of fluid samples. This instruction characteristically belongs to automated processing 84 and/or sampling manual activities 85.

In the illustrated example, the Level 3 instructions comprise further instructions 96 for product handling. This instruction characteristically belongs to sampling manual activities 85.

In the illustrated example, the Level 3 instructions comprise further instructions 97 for disposal of consumables. The consumables are of SUT type (Single Use Technology). This instruction characteristically belongs to cleaning 86.

Further, in the illustrated example, Level 2 instructions 100 comprise instructions 101 for fluid path installation, e.g. Chromatography system. This instruction characteristically belongs to material transfer and/or BOM inspection 81 and/or installation 82 and may be called upon or referenced by the Level 3 eBR as a standard operating procedure (SOP), for example defined, executed, documented and maintained by the Chromatography system and its control system or a different workflow management system.

Further, in the illustrated example, Level 2 instructions 100 comprise instructions 102 for connections of bags to chromatography system. This instruction characteristically belongs to installation 82 and/or verification 83. Again, his instruction, its execution and documentation may be called upon or referenced by the Level 3 eBR as a standard operating procedure (SOP).

Further, in the illustrated example, Level 2 instructions 100 comprise instructions 103 for execution of process automation and/or data recording. This instruction characteristically belongs to automated processing 84

Further, in the illustrated example, Level 2 instructions 100 comprise instructions 104 for documentation and/or data management. This instruction may comprise or be part of manual sampling activities 85 and/or cleaning activities 86.

This scheme for manufacture of a predetermined biopharmaceutical product is as is apparent from the above only an example. High level workflows 80 and/or instructions 90, 100 may be added or removed. The instructions may further be selected to belong to Level 2 or Level 3, whichever is appropriate. Level 2 activities may be considered as SOPs (standard operating procedures) that may be managed by a separate electronic workflow system. This Level 2 workflow management system may provide above mentioned features of instructing, guiding and correcting the operator, documenting the results, providing intelligent sensing, learning and workflow improvement capabilities. Level 2 workflow management systems may be provided by an instrument or system, such as a chromatography system and its control software or by independent stand-alone systems and software.

## Claims

1. A system (100) for monitoring manufacture of a selected biopharmaceutical product, said system comprising:
a manufacture system (110) for manufacturing a plurality of biopharmaceutical products, using at least one single-use consumable,
at least one image capture device (130) arranged to capture images of a scene comprising the manufacture system, and
a processing element (150) connected to said at least one image capture device and arranged to process images captured by said at least one image capture device to track operator interactions and/or a result of operator interactions within the scene,
wherein the processing element (150) further is arranged to
compare a predefined workflow process scheme relating to the selected biopharmaceutical product to at least a part of the tracked operator interactions with the manufacture system and/or to at least a part of the result of the tracked operator interactions with the manufacture system, and
determine whether at least one pre-set criterion set by the workflow process scheme is fulfilled based on the comparison;
wherein the predetermined workflow process scheme defines a plurality of predetermined interconnections between a plurality of first connector elements (111) of the manufacture system (110) and at least one second connector element (121) of at least one external device (120) comprising a single use consumable connectable to the manufacture system (110), and wherein the processing element (150) is arranged to process the images to identify interconnections between the first and second connector elements (111, 121) in said images, to compare each identified interconnection with the predetermined interconnections defined by the predetermined workflow process scheme and to determine which operator interactions results fulfil the at least one pre-set criterion based on said comparison so as to ensure that said at least one single-use consumable is correctly set up within said manufacture system (110).

2. The monitoring system (100) as claimed in claim 1, wherein the processing element is arranged to provide an indication that the process is verified for those operator interactions and/or for the result of those operator interactions determined to fulfil the at least one pre-set criterion set by the workflow process scheme.

3. The monitoring system (100) as claimed in claim 1 or 2, wherein the connector elements (111, 121) are provided in the form of aseptic connectors.

4. The monitoring system (100) as claimed in any preceding claim, wherein the predetermined workflow process scheme comprises characteristics of external devices connected to the manufacture system (110) for the manufacture of the selected biopharmaceutical product, and wherein the processing element (150) is arranged to
obtain information relating to at least one connected external device, compare the obtained information to characteristics of the connected external device obtained from the predetermined workflow process scheme and
determine whether the pre-set criterion set by the predetermined workflow process scheme relating to the characteristics of the external devices connected to the manufacture system is fulfilled.

5. The monitoring system (100) as claimed in claim 4, wherein: i) the processing element (150) is arranged to detect at least one of the connected external devices (120) in the image and to obtain the information relating to the at least one of the connected external devices based on the detection in the image, wherein the information may be obtained from a bar code and/or plain text on the connected external device and/or detected characteristics of the connected external device itself; or ii) the processing element (150) is arranged to obtain information relating to the at least one connected external device (120) by receiving identity data, wherein the identity data may be communicated from an RFID tag associated to the external device (120); or iii) the processing element (150) is arranged to obtain information relating to the at least one external device (120) by operator input by means of a user interface (160).

6. The monitoring system (100) as claimed in any preceding claim, wherein the at least one external device (120) further comprises a sensor element, and/or an electronic device and/or a power supply.

7. The monitoring system (100) as claimed in claim 6, wherein the processing element (150) is arranged to track operator interactions with the at least one external device, compare the tracked operator interactions with the at least one external device to the predefined workflow process scheme relating to the operator interactions with the at least one external device and determine whether the at least one pre-set criterion set by the predefined workflow process scheme is fulfilled based on said comparison, optionally wherein the processing element (150) is arranged to track the operator when connecting a respective connecting element to its corresponding external device and/or when installing the respective external device, wherein the comparison with the predetermined scheme relating to the selected biopharmaceutical product comprises comparing a procedure as determined based on the images with a procedure as indicated by the predetermined workflow process scheme relating to the selected biopharmaceutical product.

8. The monitoring system (100) as claimed in any preceding claim, further comprising at least one display element (162) arranged to display instructions for setting up for manufacture of the selected biopharmaceutical product,
wherein the processing element (150) is arranged to feed instructions to the display relating to a next or current step and/or to feed to the operator feedback information relating to the carrying out of a preceding step based on the tracking of the operator in the images; and optionally:
wherein the at least one display element (162) comprises an augmented reality display for example formed in glasses worn by the operator, or by light guide systems

9. The monitoring system (100) as claimed in any preceding claim, wherein the images of the scene captured by the at least one image capture device comprises at least in part a biopharmaceutical manufacture facility comprising the manufacture system and wherein the at least one image capture device is arranged to capture images of substantially the entire scene; and optionally:
wherein the processing element (150) is arranged to process the images captured by said at least one image capture device to determine at least one of the following:
before setting up for manufacture of the selected biopharmaceutical product is initiated, determining that the biopharmaceutical manufacturing facility is in a state ready for initiating setting up for manufacture,
whether external devices present in the facility have been moved or exchanged during setting up for manufacture or during manufacture, operator movements within the facility during setting up for manufacture or during manufacture.

10. The monitoring system (100) as claimed in any preceding claim, wherein the at least one image capture device (120) comprises a camera arranged to record images within the visual field, a thermal camera and/or a three dimensional camera, wherein at least one camera may be a video camera.

11. The monitoring system (100) as claimed in any preceding claim, further comprising a memory (170) arranged to store at least one of the following:
workflow process schemes associated to the respective biopharmaceutical product wherein the predetermined workflow associated to each product comprises at least one of the following:
data identifying which steps are to be carried out for setting up for manufacture and/or which external devices are to be connected to the manufacture system,
data identifying an order and/or timing for carrying out the steps for setting up for manufacture, for those steps this is relevant, data identifying a procedure for carrying out each step and
data identifying a configuration of interconnections between the manufacture system and external devices,
data indicating which tracked operator interactions fulfil the pre-set at least one criterion set by the workflow process scheme and/or verification data indicating which processes are verified,
images and/or annotated images captured by the at least one image capture device upon which the data indicating which tracked operator interactions fulfil the pre-set at least one criterion set by the workflow process scheme at least partly is based and/or upon which the verification data at least partly is based.

12. The monitoring system (100) as claimed in any preceding claim, where in the manufacture system comprises a liquid chromatography system preferably configured to operate with at least one column and configured for purification of a sample comprising a target product using a predefined process.

13. The monitoring system (100) as claimed in any preceding claim, wherein the manufacture system comprises at least one of the following:
a fluid transfer device,
a fluid or buffer preparation device,
a cell culturing device using bioreactor,
a cell harvest or clarification device,
a chromatography or membrane adsorption device, and
a filtration or buffer exchange device bioreactor.

14. A method (10) for monitoring a set-up for manufacture and/or setting up for manufacture and/or tearing down after manufacture of a selected biopharmaceutical products, using the monitoring system (100) as claimed in any preceding claim, said method comprising the steps of:
capturing (S1) images of a scene comprising a manufacture system (110), wherein the manufacture system (110) has capability to manufacture a plurality of biopharmaceutical products and has a plurality of first connector elements for connection to external devices,
processing said captured images (S3) to track operator interactions and/or a result of operator interactions within the scene,
comparing (S5) at least a part of tracked operator interactions and/or at least a part of the result of the tracked operator interactions with the manufacture system to a predefined workflow process scheme relating to the selected biopharmaceutical product, and
determining (S6) whether at least one pre-set criterion set by the workflow process scheme is fulfilled based on the comparison.

15. The method (10) as claimed in claim 14, wherein stored data may be used in a subsequent step wherein the manufacture process is improved (S9) based on at least semi-autonomous model based learning, whereby the production of the final biopharmaceutical product may be refined and the potential deviations in the final biopharmaceutical product may be decreased for each time the biopharmaceutical product is manufactured.

## Patentansprüche

1. System (100) zum Überwachen der Herstellung eines ausgewählten biopharmazeutischen Produkts, wobei das System umfasst
ein Herstellungssystem (110) zum Herstellen einer Vielzahl von biopharmazeutischen Produkten unter Verwendung mindestens eines Einwegverbrauchsmaterials,
mindestens eine Bilderfassungsvorrichtung (130), die angeordnet ist, um Bilder einer Szene zu erfassen, die das Herstellungssystem umfasst, und
ein Verarbeitungselement (150), das mit der mindestens einen Bilderfassungsvorrichtung verbunden und angeordnet ist, von der mindestens einen Bilderfassungsvorrichtung erfasste Bilder zu verarbeiten, um Bedienerinteraktionen und/oder ein Ergebnis von Bedienerinteraktionen innerhalb der Szene zu verfolgen,
wobei das Verarbeitungselement (150) weiter angeordnet ist zum
Vergleichen eines vordefinierten Arbeitsablauf-Prozessschemas in Bezug auf das ausgewählte biopharmazeutische Produkt mit mindestens einem Teil der verfolgten Bedienerinteraktionen mit dem Herstellungssystem und/oder mit mindestens einem Teil des Ergebnisses der verfolgten Bedienerinteraktionen mit dem Herstellungssystem und
Bestimmen, basierend auf dem Vergleich, ob mindestens ein voreingestelltes Kriterium, das durch das Arbeitsablauf-Prozessschema eingestellt wurde, erfüllt ist;
wobei das vorbestimmte Arbeitsablauf-Prozessschema eine Vielzahl von vorbestimmten Zwischenverbindungen zwischen einer Vielzahl von ersten Verbinderelementen (111) des Herstellungssystems (110) und mindestens einem zweiten Verbinderelement (121) mindestens einer externen Vorrichtung (120) definiert, umfassend ein Einwegverbrauchsmaterial, das mit dem Herstellungssystem (110) verbunden werden kann, und wobei das Verarbeitungselement (150) angeordnet ist, um die Bilder zu verarbeiten, um Zwischenverbindungen zwischen dem ersten und zweiten Verbinderelement (111, 121) in den Bildern zu identifizieren, um jede identifizierte Zwischenverbindung mit den vorbestimmten Zwischenverbindungen zu vergleichen, die durch das vorbestimmte Arbeitsablauf-Prozessschema definiert sind, und um basierend auf dem Vergleich zu bestimmen, welche Ergebnisse der Bedienerinteraktionen das mindestens eine voreingestellte Kriterium erfüllen, um sicherzustellen, dass das mindestens eine Einwegverbrauchsmaterial korrekt in das Herstellungssystem (110) aufgebaut ist.

2. Überwachungssystem (100) nach Anspruch 1, wobei das Verarbeitungselement angeordnet ist, um einen Hinweis bereitzustellen, dass der Prozess für diese Bedienerinteraktionen und/oder für das Ergebnis derjenigen Bedienerinteraktionen verifiziert ist, für welche bestimmt wurde, dass sie das mindestens eine voreingestellte Kriterium erfüllen, das durch das Arbeitsablauf-Prozessschema eingestellt wurde.

3. Überwachungssystem (100) nach Anspruch 1 oder 2, wobei die Verbinderelemente (111, 121) in Form von aseptischen Verbindern bereitgestellt sind.

4. Überwachungssystem (100) nach einem vorstehenden Anspruch, wobei das vorbestimmte Arbeitsablauf-Prozessschema Eigenschaften externer Vorrichtungen umfasst, die mit dem Herstellungssystem (110) zur Herstellung des ausgewählten biopharmazeutischen Produkts verbunden sind, und wobei das Verarbeitungselement (150) angeordnet ist zum
Erhalten von Informationen in Bezug auf mindestens eine verbundene externe Vorrichtung, Vergleichen der erhaltenen Informationen mit Eigenschaften der verbundenen externen Vorrichtung, die aus dem vorbestimmten Arbeitsablauf-Prozessschema erhalten wurden, und
Bestimmen, ob das voreingestellte Kriterium, das durch das vorbestimmte Arbeitsablauf-Prozessschema in Bezug auf die Eigenschaften der mit dem Herstellungssystem verbundenen externen Vorrichtungen eingestellt wurde, erfüllt ist.

5. Überwachungssystem (100) nach Anspruch 4, wobei: i) das Verarbeitungselement (150) angeordnet ist zum Bestimmen mindestens einer der verbundenen externen Vorrichtungen (120) im Bild und zum Erhalten von Informationen in Bezug auf die mindestens eine der verbundenen externen Vorrichtungen basierend auf der Erkennung in dem Bild, wobei die Informationen aus einem Strichcode und/oder Text auf der verbundenen externen Vorrichtung und/oder erkannten Eigenschaften der verbundenen externen Vorrichtung selbst erhalten werden können; oder ii) das Verarbeitungselement (150) angeordnet ist zum Erhalten von Informationen in Bezug auf die mindestens eine verbundene externe Vorrichtung (120) durch Empfangen von Identitätsdaten, wobei die Identitätsdaten von einem der externen Vorrichtung (120) zugeordneten RFID-Tag übermittelt werden können; oder iii) das Verarbeitungselement (150) angeordnet ist zum Erhalten von Informationen in Bezug auf die mindestens eine externe Vorrichtung (120) durch eine Bedienereingabe über eine Benutzerschnittstelle (160).

6. Überwachungssystem (100) nach einem vorstehenden Anspruch, wobei die mindestens eine externe Vorrichtung (120) weiter ein Sensorelement und/oder eine elektronische Vorrichtung und/oder eine Stromversorgung umfasst.

7. Überwachungssystem (100) nach Anspruch 6, wobei das Verarbeitungselement (150) angeordnet ist, um Bedienerinteraktionen mit der mindestens einen externen Vorrichtung zu verfolgen, die verfolgten Bedienerinteraktionen mit der mindestens einen externen Vorrichtung mit dem vordefinierten Arbeitsablauf-Prozessschema in Bezug auf die Bedienerinteraktionen mit der mindestens einen externen Vorrichtung zu vergleichen und basierend auf dem Vergleich zu bestimmen, ob das mindestens eine voreingestellte Kriterium, das durch das vordefinierte Arbeitsablauf-Prozessschema eingestellt wurde, erfüllt ist, wobei optional das Verarbeitungselement (150) angeordnet ist, um den Bediener beim Verbinden eines jeweiligen Verbindungselements mit seiner entsprechenden externen Vorrichtung und/oder beim Installieren der jeweiligen externen Vorrichtung zu verfolgen, wobei der Vergleich mit dem vorbestimmten Schema in Bezug auf das ausgewählte biopharmazeutische Produkt ein Vergleichen einer Prozedur, die basierend auf den Bildern bestimmt wurde, mit einer Prozedur wie durch das vorbestimmte Arbeitsablauf-Verarbeitungsschema in Bezug auf das ausgewählte biopharmazeutische Produkt angegeben, umfasst.

8. Überwachungssystem (100) nach einem vorstehenden Anspruch, weiter umfassend mindestens ein Anzeigeelement (162), das angeordnet ist, um Anweisungen zum Aufbauen zur Herstellung des ausgewählten biopharmazeutischen Produkts anzuzeigen,
wobei das Verarbeitungselement (150) angeordnet ist, um der Anzeige Anweisungen in Bezug auf einen nächsten oder aktuellen Schritt zuzuführen und/oder dem Bediener Feedback-Informationen in Bezug auf die Ausführung eines vorangehenden Schritts zuzuführen, basierend auf der Verfolgung des Bedieners in den Bildern; und optional:
wobei das zumindest eine Anzeigeelement (162) eine Augmented-Reality-Anzeige umfasst, die zum Beispiel in einer vom Bediener getragenen Brille oder durch Lichtleitsysteme gebildet wird.

9. Überwachungssystem (100) nach einem vorstehenden Anspruch, wobei die von der mindestens einen Bilderfassungsvorrichtung erfassten Bilder der Szene mindestens teilweise eine biopharmazeutische Herstellungsanlage umfassen, die das Herstellungssystem umfasst, und wobei die mindestens eine Bilderfassungsvorrichtung angeordnet ist, um Bilder im Wesentlichen der gesamten Szene zu erfassen; und optional:
wobei das Verarbeitungselement (150) angeordnet ist, um die von der mindestens einen Bilderfassungsvorrichtung erfassten Bilder zu verarbeiten, um mindestens eines von Folgenden zu bestimmen:
vor Beginnen eines Aufbaus zur Herstellung des ausgewählten biopharmazeutischen Produkts, Bestimmen, dass die biopharmazeutische Herstellungsanlage in einem Zustand ist, der bereit ist zum Beginnen eines Aufbaus für die Herstellung,
ob in der Anlage vorhandene externe Vorrichtungen während des Aufbauens für die Herstellung oder während der Herstellung bewegt oder ausgetauscht wurden, Bedienerbewegungen innerhalb der Anlage während des Aufbauens für die Herstellung oder während der Herstellung.

10. Überwachungssystem (100) nach einem vorstehenden Anspruch, wobei die mindestens eine Bilderfassungsvorrichtung (120) eine Kamera, die angeordnet ist, um Bilder innerhalb des Sichtfelds aufzuzeichnen, eine Wärmebildkamera und/oder eine dreidimensionale Kamera umfasst, wobei mindestens eine Kamera eine Videokamera sein kann.

11. Überwachungssystem (100) nach einem vorstehenden Anspruch, weiter umfassend einen Speicher (170), der angeordnet ist, um mindestens eines von Folgenden zu speichern:
Arbeitsablauf-Prozessschemata, die dem jeweiligen biopharmazeutischen Produkt zugeordnet sind, wobei der vorbestimmte Arbeitsablauf, der jedem Produkt zugeordnet ist, mindestens eines von Folgendem umfasst:
Daten, die identifizieren, welche Schritte zum Aufbauen zur Herstellung auszuführen sind und/oder welche externen Vorrichtungen mit dem Herstellungssystem zu verbinden sind,
Daten, die eine Reihenfolge und/oder einen Zeitpunkt für die Ausführung der Schritte zum Aufbauen zur Herstellung identifizieren, für diese Schritte dies relevant ist,
Daten, die eine Prozedur zum Ausführen jedes Schritts identifizieren, und
Daten, die eine Konfiguration von Zwischenverbindungen zwischen dem Herstellungssystem und den externen Vorrichtungen identifizieren,
Daten, die angeben, welche verfolgten Bedienerinteraktionen das voreingestellte mindestens eine Kriterium erfüllen, das durch das Arbeitsablauf-Prozessschema eingestellt wurde und/oder Verifizierungsdaten, die angeben, welche Prozesse verifiziert sind,
Bilder und/oder kommentierte Bilder, die von der mindestens einen Bilderfassungsvorrichtung erfasst wurden, auf denen die Daten mindestens teilweise basieren, die angeben, welche verfolgten Bedienerinteraktionen das voreingestellte mindestens eine durch das Arbeitsablauf-Prozessschema eingestellte Kriterium erfüllen, und/oder auf denen die Verifizierungsdaten mindestens teilweise basieren.

12. Überwachungssystem (100) nach einem vorstehenden Anspruch, wobei das Herstellungssystem ein Flüssigkeitschromatographiesystem umfasst, das vorzugsweise für den Betrieb mit mindestens einer Säule konfiguriert ist und für eine Reinigung einer Probe, die ein Zielprodukt umfasst, unter Verwendung eines vordefinierten Prozesses konfiguriert ist.

13. Überwachungssystem (100) nach einem vorstehenden Anspruch, wobei das Herstellungssystem mindestens eines von Folgendem umfasst:
eine Fluidtransfervorrichtung,
eine Fluid- oder Pufferzubereitungsvorrichtung,
eine Zellkultivierungsvorrichtung unter Verwendung eines Bioreaktors,
eine Zellernte- oder -klärvorrichtung,
eine Chromatographie- oder Membranadsorptionsvorrichtung und
einen Filtrations- oder Pufferaustauschvorrichtungs-Bioreaktor.

14. Verfahren (10) zum Überwachen eines Aufbaus für die Herstellung und/oder eines Aufbauens für die Herstellung und/oder eines Abbaus nach der Herstellung eines ausgewählten biopharmazeutischen Produkts unter Verwendung des Überwachungssystems (100) nach einem vorstehenden Anspruch, wobei das Verfahren die folgenden Schritte umfasst:
Erfassen (S1) von Bildern einer Szene, die ein Herstellungssystem (110) umfasst, wobei das Herstellungssystem (110) dazu fähig ist, eine Vielzahl biopharmazeutischer Produkte herzustellen und eine Vielzahl von ersten Verbinderelementen zum Verbinden mit externen Vorrichtungen aufweist,
Verarbeiten der erfassten Bilder (S3), um Bedienerinteraktionen und/oder ein Ergebnis von Bedienerinteraktionen innerhalb der Szene zu verfolgen,
Vergleichen (S5) mindestens eines Teils von verfolgten Bedienerinteraktionen und/oder mindestens eines Teils des Ergebnisses der verfolgten Bedienerinteraktionen mit dem Herstellungssystem mit einem vordefinierten Arbeitsablauf-Prozessschema in Bezug auf das ausgewählte biopharmazeutische Produkt und
Bestimmen (S6), ob mindestens ein voreingestelltes Kriterium, das durch das Arbeitsablauf-Prozessschema eingestellt wurde, basierend auf dem Vergleich erfüllt ist.

15. Verfahren (10) nach Anspruch 14, wobei gespeicherte Daten in einem nachfolgenden Schritt verwendet werden können, wobei der Herstellungsprozess basierend auf mindestens halbautonomem, modellbasierten Lernen verbessert wird (S9), wodurch die Produktion des biopharmazeutischen Endprodukts verfeinert werden kann und die potenziellen Abweichungen im biopharmazeutischen Endprodukt bei jeder Herstellung des biopharmazeutischen Produkts verringert werden können.

## Revendications

1. Système (100) pour surveiller la fabrication d'un produit biopharmaceutique sélectionné, ledit système comprenant
un système de fabrication (110) pour fabriquer une pluralité de produits biopharmaceutiques, à l'aide d'au moins un consommable à usage unique,
au moins un dispositif de capture d'image (130) agencé pour capturer des images d'une scène comprenant le système de fabrication, et
un élément de traitement (150) raccordé au dit au moins un dispositif de capture d'image et agencé pour traiter des images capturées par ledit au moins un dispositif de capture d'image afin de suivre des interactions d'opérateur et/ou un résultat d'interactions d'opérateur dans la scène,
dans lequel l'élément de traitement (150) est en outre agencé pour
comparer un schéma de processus de flux de travail prédéfini se rapportant au produit biopharmaceutique sélectionné à au moins une partie des interactions d'opérateur suivies avec le système de fabrication et/ou à au moins une partie du résultat des interactions d'opérateur suivies avec le système de fabrication, et
déterminer si au moins un critère prédéfini défini par le schéma de processus de flux de travail est satisfait sur la base de la comparaison ;
dans lequel le schéma de processus de flux de travail prédéterminé définit une pluralité d'interconnexions prédéterminées entre une pluralité de premiers éléments de connecteur (111) du système de fabrication (110) et au moins un second élément de connecteur (121) d'au moins un dispositif externe (120) comprenant un consommable à usage unique pouvant être raccordé au système de fabrication (110) et dans lequel l'élément de traitement (150) est agencé pour traiter les images afin d'identifier des interconnexions entre les premier et second éléments de connecteur (111, 121) dans lesdites images, pour comparer chaque interconnexion identifiée avec les interconnexions prédéterminées définies par le schéma de processus de flux de travail prédéterminé et pour déterminer quels résultats d'interactions d'opérateur satisfont le au moins un critère prédéfini sur la base de ladite comparaison de sorte à garantir que ledit au moins un consommable à usage unique est correctement configuré dans ledit système de fabrication (110).

2. Système de surveillance (100) selon la revendication 1, dans lequel l'élément de traitement est agencé pour fournir une indication que le processus est vérifié pour ces interactions d'opérateur et/ou pour le résultat de ces interactions d'opérateur déterminées pour satisfaire le au moins un critère prédéfini défini par le schéma de processus de flux de travail.

3. Système de surveillance (100) selon la revendication 1 ou 2, dans lequel les éléments de connecteur (111, 121) sont prévus sous la forme de connecteurs aseptiques.

4. Système de surveillance (100) selon une quelconque revendication précédente, dans lequel le schéma de processus de flux de travail prédéterminé comprend des caractéristiques de dispositifs externes raccordés au système de fabrication (110) pour la fabrication du produit biopharmaceutique sélectionné et dans lequel l'élément de traitement (150) est arrangé pour
obtenir des informations se rapportant à au moins un dispositif externe raccordé, comparer les informations obtenues à des caractéristiques du dispositif externe raccordé obtenues à partir du schéma de processus de flux de travail prédéterminé et
déterminer si le critère prédéfini défini par le schéma de processus de flux de travail prédéterminé se rapportant aux caractéristiques des dispositifs externes raccordés au système de fabrication est satisfait.

5. Système de surveillance (100) selon la revendication 4, dans lequel : i) l'élément de traitement (150) est agencé pour détecter au moins un des dispositifs externes raccordés (120) dans l'image et pour obtenir les informations se rapportant à le au moins un des dispositifs externes raccordés sur la base de la détection dans l'image, dans lequel les informations peuvent être obtenues à partir d'un code à barres et/ou d'un texte en clair sur le dispositif externe raccordé et/ou de caractéristiques détectées du dispositif externe raccordé lui-même ; ou ii) l'élément de traitement (150) est agencé pour obtenir des informations se rapportant à le au moins un dispositif externe raccordé (120) en recevant des données d'identité, dans lequel les données d'identité peuvent être communiquées à partir d'une étiquette RFID associée au dispositif externe (120) ; ou iii) l'élément de traitement (150) est agencé pour obtenir des informations se rapportant à le au moins un dispositif externe (120) par une entrée d'opérateur au moyen d'une interface utilisateur (160).

6. Système de surveillance (100) selon une quelconque revendication précédente, dans lequel le au moins un dispositif externe (120) comprend en outre un élément capteur et/ou un appareil électronique et/ou une alimentation électrique.

7. Système de surveillance (100) selon la revendication 6, dans lequel l'élément de traitement (150) est agencé pour suivre des interactions d'opérateur avec le au moins un dispositif externe, comparer les interactions d'opérateur suivies avec le au moins un dispositif externe au schéma de processus de flux de travail prédéfini se rapportant aux interactions d'opérateur avec le au moins un dispositif externe et déterminer si le au moins un critère prédéfini défini par le schéma de processus de flux de travail prédéfini est satisfait sur la base de ladite comparaison, facultativement dans lequel l'élément de traitement (150) est agencé pour suivre l'opérateur lors du raccordement d'un élément de raccordement respectif à son dispositif externe correspondant et/ou lors de l'installation du dispositif externe respectif, dans lequel la comparaison avec le schéma prédéterminé se rapportant au produit biopharmaceutique sélectionné comprenant la comparaison d'une procédure telle que déterminée sur la base des images avec une procédure telle qu'indiquée par le schéma de processus de flux de travail prédéterminé se rapportant au produit biopharmaceutique sélectionné.

8. Système de surveillance (100) selon une quelconque revendication précédente, comprenant en outre au moins un élément d'affichage (162) agencé pour afficher des instructions pour la préparation de la fabrication du produit biopharmaceutique sélectionné,
dans lequel l'élément de traitement (150) est agencé pour fournir des instructions à l'affichage se rapportant à une étape suivante ou en cours et/ou fournir à l'opérateur des informations de retour se rapportant à la réalisation d'une étape précédente basée sur le suivi de l'opérateur dans les images ; et facultativement :
dans lequel le au moins un élément d'affichage (162) comprend un affichage à réalité augmentée, par exemple formé dans des lunettes portées par l'opérateur, ou par des systèmes de guidage de lumière

9. Système de surveillance (100) selon une quelconque revendication précédente, dans lequel les images de la scène capturée par le au moins un dispositif de capture d'image comprend au moins en partie une installation de fabrication biopharmaceutique comprenant le système de fabrication et dans lequel le au moins un dispositif de capture d'image est agencé pour capturer des images de sensiblement la totalité de la scène ; et facultativement :
dans lequel l'élément de traitement (150) est agencé pour traiter les images capturées par ledit au moins un dispositif de capture d'image afin de déterminer au moins l'un des éléments suivants :
avant que la préparation de la fabrication du produit biopharmaceutique sélectionné soit lancée, la détermination que l'installation de fabrication biopharmaceutique est dans un état prêt à initier la préparation de la fabrication,
si des dispositifs externes présents dans l'installation ont été déplacés ou échangés lors de la mise en fabrication ou pendant la fabrication, des mouvements d'opérateur dans l'installation pendant la préparation de la fabrication ou pendant la fabrication.

10. Système de surveillance (100) selon une quelconque revendication précédente, dans lequel le au moins un dispositif de capture d'image (120) comprend une caméra agencée pour enregistrer des images dans le champ visuel, une caméra thermique et/ou une caméra tridimensionnelle, dans lequel au moins une caméra pouvant être une caméra vidéo.

11. Système de surveillance (100) selon une quelconque revendication précédente, comprenant en outre une mémoire (170) agencée pour stocker au moins l'un des éléments suivants :
des schémas de processus de flux de travail associés au produit biopharmaceutique respectif, dans lequel le flux de travail prédéterminé associé à chaque produit comprend au moins l'un des éléments suivants :
des données identifiant quelles étapes doivent être effectuées pour la préparation de la fabrication et/ou quels appareils externes doivent être raccordés au système de fabrication,
des données identifiant une commande et/ou un calendrier pour exécuter les étapes de préparation de la fabrication, pour ces étapes, cela est pertinent,
des données identifiant une procédure pour réaliser chaque étape et
des données identifiant une configuration d'interconnexions entre le système de fabrication et des dispositifs externes,
des données indiquant quelles interactions d'opérateur suivies satisfont le au moins un critère prédéfini défini par le schéma de processus de flux de travail et/ou des données de vérification indiquant quels processus sont vérifiés,
des images et/ou des images annotées capturées par le au moins un dispositif de capture d'image sur lequel les données indiquant quelles interactions d'opérateur suivies satisfont le au moins un critère prédéfini défini par le schéma de processus de flux de travail sont au moins partiellement basées et/ou sur lesquelles les données de vérification sont au moins partiellement basées.

12. Système de surveillance (100) selon une quelconque revendication précédente, dans lequel le système de fabrication comprend un système de chromatographie liquide configuré, de préférence, pour fonctionner avec au moins une colonne et configuré pour la purification d'un échantillon comprenant un produit cible à l'aide d'un processus prédéfini.

13. Système de surveillance (100) selon une quelconque revendication précédente, dans lequel le système de fabrication comprend au moins l'un des éléments suivants :
un dispositif de transfert de fluide,
un dispositif de préparation de fluide ou de tampon,
un dispositif de culture cellulaire utilisant un bioréacteur,
un dispositif de récolte ou de clarification de cellules,
un dispositif de chromatographie ou d'adsorption sur membrane, et
un bioréacteur à dispositif de filtration ou d'échange de tampon.

14. Procédé (10) de surveillance d'une configuration pour la fabrication et/ou la préparation pour la fabrication et/ou le démontage après fabrication d'un produit biopharmaceutique sélectionné, à l'aide du système de surveillance (100) selon une quelconque revendication précédente, ledit procédé comprenant les étapes consistant :
à capturer (S1) des images d'une scène comprenant un système de fabrication (110), dans lequel le système de fabrication (110) présente la capacité de fabriquer une pluralité de produits biopharmaceutiques et présente une pluralité de premiers éléments de connecteur pour un raccordement à des dispositifs externes,
à traiter lesdites images capturées (S3) pour suivre des interactions d'opérateur et/ou un résultat d'interactions d'opérateur dans la scène,
à comparer (S5) au moins une partie des interactions d'opérateur suivies et/ou au moins une partie du résultat des interactions d'opérateur suivies avec le système de fabrication vers un schéma de processus de flux de travail prédéfini se rapportant au produit biopharmaceutique sélectionné, et
à déterminer (S6) si au moins un critère prédéfini défini par le schéma de processus de flux de travail est satisfait sur la base de la comparaison.

15. Procédé (10) selon la revendication 14, dans lequel des données stockées peuvent être utilisées dans une étape ultérieure dans laquelle le processus de fabrication est amélioré (S9) sur la base d'un apprentissage basé sur un modèle au moins semi-autonome, selon lequel la production du produit biopharmaceutique final peut être affinée et les écarts potentiels dans le produit biopharmaceutique final peuvent être réduits à chaque fois que le produit biopharmaceutique est fabriqué.
